# EUROPEAN PATENT SPECIFICATION

(11) **EP 2 296 558 B1**
(45) Date of publication and mention of the grant of the patent: **19.08.2020**
(21) Application number: 09763167.5
(22) Date of filing: 16.05.2009
(51) Int. Cl.: A61B 90/90, A61B 90/92, A61B 90/94, A61B 17/06, A61B 17/04

(54) **CODED HETEROFUNCTIONAL SUTURES AND FORMING METHODS**
KODIERTES HETEROFUNKTIONALES NAHTMATERIAL UND VERFAHREN ZUR HERSTELLUNG DES NAHTMATERIALS
SUTURES HÉTÉROFONCTIONNELLES CODÉES ET PROCÉDÉS DE FABRICATION

(30) Priority: 16.05.2008 US 53912 P
(43) Date of publication of application: 23.03.2011
(73) Proprietor: Ethicon LLC, Guaynabo 00969 (PR)
(72) Inventor: AVELAR, Rui, Vancouver British Columbia V6J 2W7 (CA); GORALTCHOUK, Alexei, Richmond British Columbia V6Y 3Z1 (CA); HERRMANN, Robert, A., Vancouver British Columbia V6B 1X9 (CA); LUSCOMBE, Brian, H., Basking Ridge New Jersey 07920 (US); D'AGOSTINO, William, L., Hamden Connecticut 06518 (US)
(74) Representative: Small, Gary James
(86) International application number: PCT/US2009/044274
(87) International publication number: WO 2009/151876

(56) References cited:
- US-A- 3 762 418
- US-A- 5 454 834
- US-A1- 2003 149 447
- US-A1- 2003 149 447
- US-A1- 2007 005 110
- US-A1- 2007 225 763
- US-A1- 2008 077 181
- US-A1- 2008 082 113

## Description

### TECHNICAL FIELD

The present disclosure relates generally to filaments for surgical procedures, methods of manufacturing filaments for surgical procedures, and uses thereof.

### BACKGROUND

Wound closure devices such as sutures, staples and tacks have been widely used in superficial and deep surgical procedures in humans and animals for closing wounds, repairing traumatic injuries or defects, joining tissues together (bringing severed tissues into approximation, closing an anatomical space, affixing single or multiple tissue layers together, creating an anastomosis between two hollow/luminal structures, adjoining tissues, attaching or reattaching tissues to their proper anatomical location), attaching foreign elements to tissues (affixing medical implants, devices, prostheses and other functional or supportive devices), and for repositioning tissues to new anatomical locations (repairs, tissue elevations, tissue grafting and related procedures) to name but a few examples.

Sutures are often used as wound closure devices. Sutures typically consist of a filamentous suture thread attached to a needle with a sharp point. Suture threads can be made from a wide variety of materials including bioabsorbable (i.e., that break down completely in the body over time), or non-absorbable (permanent; non-degradable) materials. Absorbable sutures have been found to be particularly useful in situations where suture removal might jeopardize the repair or where the natural healing process renders the support provided by the suture material unnecessary after wound healing has been completed; as in, for example, completing an uncomplicated skin closure. Non-degradable (non- absorbable) sutures are used in wounds where healing may be expected to be protracted or where the suture material is needed to provide physical support to the wound for long periods of time; as in, for example, deep tissue repairs, high tension wounds, many orthopedic repairs and some types of surgical anastomosis. Also, a wide variety of surgical needles are available, and the shape, and size of the needle body and the configuration of the needle tip is typically selected based upon the needs of the particular application.

To use an ordinary suture, the suture needle is advanced through the desired tissue on one side of the wound and then through the adjacent side of the wound. The suture is then formed into a "loop" which is completed by tying a knot in the suture to hold the wound closed. Knot tying takes time and causes a range of complications, including, but not limited to (i) spitting (a condition where the suture, usually a knot) pushes through the skin after a subcutaneous closure), (ii) infection (bacteria are often able to attach and grow in the spaces created by a knot), (iii) bulk/mass (a significant amount of suture material left in a wound is the portion that comprises the knot), (iv) slippage (knots can slip or come untied), and (v) irritation (knots serve as a bulk "foreign body" in a wound). Suture loops associated with knot tying may lead to ischemia (knots can create tension points that can strangulate tissue and limit blood flow to the region) and increased risk of dehiscence or rupture at the surgical wound. Knot tying is also labor intensive and can comprise a significant percentage of the time spent closing a surgical wound. Additional operative procedure time is not only bad for the patient (complication rates rise with time spent under anesthesia), but it also adds to the overall cost of the operation (many surgical procedures are estimated to cost between $15 and $30 per minute of operating time).

Self-retaining sutures (including barbed sutures) differ from conventional sutures in that self-retaining sutures possess numerous tissue retainers (such as barbs) which anchor the self-retaining suture into the tissue following deployment and resist movement of the suture in a direction opposite to that in which the retainers face, thereby eliminating the need to tie knots to affix adjacent tissues together (a "knotless" closure). Knotless tissue-approximating devices having barbs have been previously described in, for example, U.S. Pat. No. 5,374,268, disclosing armed anchors having barb-like projections, while suture assemblies having barbed lateral members have been described in U.S. Pat. Nos. 5,584,859 and 6,264,675. Sutures having a plurality of barbs positioned along a greater portion of the suture are described in U.S. Pat No. 5,931,855, which discloses a unidirectional barbed suture, and U.S. Pat. No. 6,241,747, which discloses a bidirectional barbed suture. Methods and apparatus for forming barbs on sutures have been described in, for example, U.S. Pat. Nos. 6,848,152. Self-retaining systems for wound closure also result in better approximation of the wound edges, evenly distribute the tension along the length of the wound (reducing areas of tension that can break or lead to ischemia), decrease the bulk of suture material remaining in the wound (by eliminating knots) and reduce spitting (the extrusion of suture material - typically knots - through the surface of the skin. All of these features are thought to reduce scarring, improve cosmesis, and increase wound strength relative to wound closures using plain sutures or staples. Thus, self-retaining sutures, because such sutures avoid knot tying, allow patients to experience an improved clinical outcome, and also save time and costs associated with extended surgeries and follow-up treatments.

The ability of self-retaining sutures to anchor and hold tissues in place even in the absence of tension applied to the suture by a knot is a feature that also provides superiority over plain sutures. When closing a wound that is under tension, this advantage manifests itself in several ways: (i) self-retaining sutures have a multiplicity of retainers which can dissipate tension along the entire length of the suture (providing hundreds of "anchor" points this produces a superior cosmetic result and lessens the chance that the suture will "slip" or pull through) as opposed to knotted interrupted sutures which concentrate the tension at discrete points; (ii) complicated wound geometries can be closed (circles, arcs, jagged edges) in a uniform manner with more precision and accuracy than can be achieved with interrupted sutures; (iii) self-retaining sutures eliminate the need for a "third hand" which is often required for maintaining tension across the wound during traditional suturing and knot tying (to prevent "slippage" when tension is momentarily released during tying); (iv) self-retaining sutures are superior in procedures where knot tying is technically difficult, such as in deep wounds or laparoscopic/endoscopic procedures; and (v) self-retaining sutures can be used to approximate and hold the wound prior to definitive closure. As a result, self-retaining sutures provide easier handling in anatomically tight or deep places (such as the pelvis, abdomen and thorax) and make it easier to approximate tissues in laparoscopic/endoscopic and minimally invasive procedures; all without having to secure the closure via a knot. Greater accuracy allows self-retaining sutures to be used for more complex closures (such as those with diameter mismatches, larger defects or purse string suturing) than can be accomplished with plain sutures.

A self-retaining suture may be unidirectional, having one or more retainers oriented in one direction along the length of the suture thread; or bidirectional, typically having one or more retainers oriented in one direction along a portion of the thread, followed by one or more retainers oriented in another (often opposite) direction over a different portion of the thread (as described with barbed retainers in U.S. Pat. Nos. 5,931,855 and. 6,241,747). Although any number of sequential or intermittent configurations of retainers are possible, a common form of bidirectional self-retaining suture involves a needle at one end of a suture thread which has barbs having tips projecting "away" from the needle until the transition point (often the midpoint) of the suture is reached; at the transition point the configuration of barbs reverses itself about 180° (such that the barbs are now facing in the opposite direction) along the remaining length of the suture thread before attaching to a second needle at the opposite end (with the result that the barbs on this portion of the suture also have tips projecting "away" from the nearest needle). Projecting "away" from the needle means that the tip of the barb is further away from the needle and the portion of suture comprising the barb may be pulled more easily through tissue in the direction of the needle than in the opposite direction. Put another way, the barbs on both "halves" of a typical bidirectional self-retaining suture have tips that point towards the middle, with a transition segment (lacking barbs) interspersed between them, and with a needle attached to either end.

US 2003/149447 A1 discloses a double-ended, symmetrical, barbed surgical suture having a mark to indicate the centre of the suture between two barbed portions and/or depth markings on the suture body.

US 2008/082113 A1 discloses a suture which has a core and can be partially or completely surrounded by a like or different material which forms barbs. The suture can have identifiers in the form of colour coding where the colour is used to indicate regions of distinctive mechanical properties, or indicate regions with or without barbs. Alternatively, markers are for visualisation when using radiographic imaging modalities.

### BRIEF SUMMARY OF INVENTION

Despite the multitude of advantages of unidirectional and bidirectional self-retaining sutures, there remains a need to improve upon the design of the suture such that a variety of limitations can be eliminated and enhanced and/or additional functionality is provided.

The self-retaining suture according to the invention is defined in claim 1. The method of forming a self-retaining suture of the invention is defined in claim 7. Preferred embodiments are defined in the dependent claims.

### DESCRIPTION OF DRAWINGS

Features of the invention, its nature and various advantages will be apparent from the accompanying drawings and the following detailed description of various embodiments.
FIG. 1A is a perspective view of a bidirectional self-retaining suture.
FIGS. 1B-1D are enlarged views of portions of the suture of FIG. 1A;
FIG. 1E illustrates an endoscopic view of a bidirectional self-retaining.
FIGS. 2A-2D show examples of heterofunctional sutures having two or more sections having different features.
FIGS. 2E-2F show examples of an application of a heterofunctional suture such as the heterofunctional suture of FIG. 2B.
FIGS. 3A-31 show examples of suture markers which may be associated with one or more sections of a suture having different features according to embodiments of the present invention.
FIGS. 4A-4E show examples of needle and pledget markers which may be associated with one or more sections of a suture having different features according to embodiments of the present invention.
FIGS. 5A-5H show examples of suture markers and needle markers which may be associated with a suture to indicate a condition of a suture, such as tension, according to embodiments of the present invention.
FIGS. 6A-6D show examples of active suture markers which may be associated with one or more sections of a suture to indicate features and/or conditions of the suture according to embodiments of the present invention.
FIG. 7A shows an endoscopic system capable of utilizing machine- readable suture markers and needle markers associated with one or more sections of a suture to indicate a fixed feature or condition of a suture according to an embodiment of the present invention.
FIG. 7B shows a flowchart showing steps in the operation of a videoscopic system capable of utilizing machine-readable suture markers and needle markers associated with a suture to indicate a fixed feature or condition of a suture according to an embodiment of the present invention.
FIG. 7C shows an example of a video display augmented with suture information derived from suture markers according to an embodiment of the present invention.
FIG. 8 is a partial elevation view of a self-retaining suture having a transition segment deformation.
FIGS. 9A and 9B are partial elevation views of self-retaining sutures having transition segment indentations.
FIGS. 10A and 10B are partial elevation views of self-retaining sutures having transition segment relief forms.

### DETAILED DESCRIPTION

### DEFINITIONS

Definitions of certain terms that may be used hereinafter include the following.

"Self-retaining system" refers to a self-retaining suture together with devices for deploying the suture into tissue. Such deployment devices include, without limitation, suture needles and other deployment devices as well as sufficiently rigid and sharp ends on the suture itself to penetrate tissue.

"Self-retaining suture" refers to a suture that comprises features on the suture filament for engaging tissue without the need for a knot or suture anchor.

"Tissue retainer" (or simply "retainer") or "barb" refers to a physical feature of a suture filament which is adapted to mechanically engage tissue and resist movement of the suture in at least one axial directions. By way of example only, tissue retainer or retainers can include hooks, projections, barbs, darts, extensions, bulges, anchors, protuberances, spurs, bumps, points, cogs, tissue engagers, traction devices, surface roughness, surface irregularities, surface defects, edges, facets and the like. In certain configurations, tissue retainers are adapted to engage tissue to resist movement of the suture in a direction other than the direction in which the suture is deployed into the tissue by the physician, by being oriented to substantially face the deployment direction. In some embodiments the retainers lie flat when pulled in the deployment direction and open or "fan out" when pulled in a direction contrary to the deployment direction. As the tissue-penetrating end of each retainer faces away from the deployment direction when moving through tissue during deployment, the tissue retainers should not catch or grab tissue during this phase. Once the self-retaining suture has been deployed, a force exerted in another direction (often substantially opposite to the deployment direction) causes the retainers to be displaced from the deployment position (i.e. resting substantially along the suture body), forces the retainer ends to open (or "fan out") from the suture body in a manner that catches and penetrates into the surrounding tissue, and results in tissue being caught between the retainer and the suture body; thereby "anchoring" or affixing the self-retaining suture in place. In certain other embodiments, the tissue retainers may be configured to permit motion of the suture in one direction and resist movement of the suture in another direction without fanning out or deploying. In certain other configurations, the tissue retainer may be configured or combined with other tissue retainers to resist motion of the suture filament in both directions. Typically a suture having such retainers is deployed through a device such as a cannula which prevents contact between the retainers and the tissue until the suture is in the desired location.

"Retainer configurations" refers to configurations of tissue retainers and can include features such as size, shape, flexibility, surface characteristics, and so forth. These are sometimes also referred to as "barb configurations".

"Bidirectional suture" refers to a self-retaining suture having retainers oriented in one direction at one end and retainers oriented in the other direction at the other end. A bidirectional suture is typically armed with a needle at each end of the suture thread. Many bidirectional sutures have a transition segment located between the two barb orientations.

"Transition segment" refers to a retainer-free (barb-free) portion of a bidirectional suture located between a first set of retainers (barbs) oriented in one direction and a second set of retainers (barbs) oriented in another direction. The transition segment can be at about the midpoint of the self-retaining suture, or closer to one end of the self-retaining suture to form an asymmetrical self-retaining suture system.

"Suture thread" refers to the filamentary body component of the suture. The suture thread may be a monofilament, or comprise multiple filaments as in a braided suture. The suture thread may be made of any suitable biocompatible material, and may be further treated with any suitable biocompatible material, whether to enhance the sutures' strength, resilience, longevity, or other qualities, or to equip the sutures to fulfill additional functions besides joining tissues together, repositioning tissues, or attaching foreign elements to tissues.

"Monofilament suture" refers to a suture comprising a monofilamentary suture thread.

"Braided suture" refers to a suture comprising a multifilamentary suture thread. The filaments in such suture threads are typically braided, twisted, or woven together.

"Degradable suture" (also referred to as "biodegradable suture" or "absorbable suture") refers to a suture which, after introduction into a tissue is broken down and absorbed by the body. Typically, the degradation process is at least partially mediated by, or performed in, a biological system. "Degradation" refers to a chain scission process by which a polymer chain is cleaved into oligomers and monomers. Chain scission may occur through various mechanisms, including, for example, by chemical reaction (e.g., hydrolysis, oxidation/reduction, enzymatic mechanisms or a combination of these) or by a thermal or photolytic process. Polymer degradation may be characterized, for example, using gel permeation chromatography (GPC), which monitors the polymer molecular mass changes during erosion and breakdown. Degradable suture material may include polymers such as polyglycolic acid, copolymers of glycolide and lactide, copolymers of trimethylene carbonate and glycolide with diethylene glycol (e.g., MAXONTM, Tyco Healthcare Group), terpolymer composed of glycolide, trimethylene carbonate, and dioxanone (e.g., BIOSYNTM [glycolide (60%), trimethylene carbonate (26%), and dioxanone (14%)], Tyco Healthcare Group), copolymers of glycolide, caprolactone, trimethylene carbonate, and lactide (e.g., CAPROSYNTM, Tyco Healthcare Group). A dissolvable suture can also include partially deacetylated polyvinyl alcohol. Polymers suitable for use in degradable sutures can be linear polymers, branched polymers or multi-axial polymers. Examples of multi-axial polymers used in sutures are described in U.S. Patent Application Publication Nos. 20020161168, 20040024169, and 20040116620. Sutures made from degradable suture material lose tensile strength as the material degrades. Degradable sutures can be in either a braided multifilament form or a monofilament form.

"Non-degradable suture" (also referred to as "non-absorbable suture") refers to a suture comprising material that is not degraded by chain scission such as chemical reaction processes (e.g., hydrolysis, oxidation/reduction, enzymatic mechanisms or a combination of these) or by a thermal or photolytic process. Non-degradable suture material includes polyamide (also known as nylon, such as nylon 6 and nylon 6,6), polyester (e.g., polyethylene terephthlate), polytetrafluoroethylene (e.g., expanded polytetrafluoroethylene), polyether-ester such as polybutester (block copolymer of butylene terephthalate and polytetra methylene ether glycol), polyurethane, metal alloys, metal (e.g., stainless steel wire), polypropylene, polyethelene, silk, and cotton. Sutures made of non-degradable suture material are suitable for applications in which the suture is meant to remain permanently or is meant to be physically removed from the body.

"Suture diameter" refers to the diameter of the body of the suture. It is to be understood that a variety of suture lengths may be used with the sutures described herein and that while the term "diameter" is often associated with a circular periphery, it is to be understood herein to indicate a cross-sectional dimension associated with a periphery of any shape. Suture sizing is based upon diameter. United States Pharmacopeia ("USP") designation of suture size runs from 0 to 7 in the larger range and 1-0 to 11-0 in the smaller range; in the smaller range, the higher the value preceding the hyphenated zero, the smaller the suture diameter. The actual diameter of a suture will depend on the suture material, so that, by way of example, a suture of size 5-0 and made of collagen will have a diameter of 0.15 mm, while sutures having the same USP size designation but made of a synthetic absorbable material or a non-absorbable material will each have a diameter of 0.1 mm. The selection of suture size for a particular purpose depends upon factors such as the nature of the tissue to be sutured and the importance of cosmetic concerns; while smaller sutures may be more easily manipulated through tight surgical sites and are associated with less scarring, the tensile strength of a suture manufactured from a given material tends to decrease with decreasing size. It is to be understood that the sutures and methods of manufacturing sutures disclosed herein are suited to a variety of diameters, including without limitation 7, 6, 5, 4, 3, 2, 1, 0, 1-0, 2-0, 3-0, 4-0, 5-0, 6-0, 7-0, 8-0, 9-0, 10-0 and 11-0.

"Needle attachment" refers to the attachment of a needle to a suture requiring same for deployment into tissue, and can include methods such as crimping, swaging, using adhesives, and so forth. The suture thread is attached to the suture needle using methods such as crimping, swaging and adhesives. Attachment of sutures and surgical needles is described in U.S. Patent Nos. 3,981,307, 5,084,063, 5,102,418, 5,123,911, 5,500,991, 5,722,991, 6,012,216, and 6,163,948, and U.S. Patent Application Publication No. US 2004/0088003). The point of attachment of the suture to the needle is known as the swage.

"Suture needle" refers to needles used to deploy sutures into tissue, which come in many different shapes, forms and compositions. There are two main types of needles, traumatic needles and atraumatic needles. Traumatic needles have channels or drilled ends (that is, holes or eyes) and are supplied separate from the suture thread and are threaded on site. Atraumatic needles are eyeless and are attached to the suture at the factory by swaging or other methods whereby the suture material is inserted into a channel at the blunt end of the needle which is then deformed to a final shape to hold the suture and needle together. As such, atraumatic needles do not require extra time on site for threading and the suture end at the needle attachment site is generally smaller than the needle body. In the traumatic needle, the thread comes out of the needle's hole on both sides and often the suture rips the tissues to a certain extent as it passes through. Most modern sutures are swaged atraumatic needles. Atraumatic needles may be permanently swaged to the suture or may be designed to come off the suture with a sharp straight tug. These "pop-offs" are commonly used for interrupted sutures, where each suture is only passed once and then tied. For barbed sutures that are uninterrupted, these atraumatic needles are preferred.

Suture needles may also be classified according to the geometry of the tip or point of the needle. For example, needles may be (i) "tapered" whereby the needle body is round and tapers smoothly to a point; (ii) "cutting" whereby the needle body is triangular and has a sharpened cutting edge on the inside; (iii) "reverse cutting" whereby the cutting edge is on the outside; (iv) "trocar point" or "taper cut" whereby the needle body is round and tapered, but ends in a small triangular cutting point; (v) "blunt" points for sewing friable tissues; (vi) "side cutting" or "spatula points" whereby the needle is flat on top and bottom with a cutting edge along the front to one side (these are typically used for eye surgery).

Suture needles may also be of several shapes including, (i) straight, (ii) half curved or ski, (iii) 1/4 circle, (iv) 3/8 circle, (v) 1/2 circle, (vi) 5/8 circle, (v) and compound curve.

Suturing needles are described, for example, in US Patent Nos. 6,322,581 and 6,214,030 (Mani, Inc., Japan); and 5,464,422 (W.L. Gore, Newark, DE); and 5,941,899; 5,425,746; 5,306,288 and 5,156,615 (US Surgical Corp., Norwalk, CT); and 5,312,422 (Linvatec Corp., Largo, FL); and 7,063,716 (Tyco Healthcare, North Haven, CT). Other suturing needles are described, for example, in US Patent Nos. 6,129,741; 5,897,572; 5,676,675; and 5,693,072. The sutures described herein may be deployed with a variety of needle types (including without limitation curved, straight, long, short, micro, and so forth), needle cutting surfaces (including without limitation, cutting, tapered, and so forth), and needle attachment techniques (including without limitation, drilled end, crimped, and so forth). Moreover, the sutures described herein may themselves include sufficiently rigid and sharp ends so as to dispense with the requirement for deployment needles altogether.

"Needle diameter" refers to the diameter of a suture deployment needle at the widest point of that needle. While the term "diameter" is often associated with a circular periphery, it is to be understood herein to indicate a cross-sectional dimension associated with a periphery of any shape.

"Armed suture" refers to a suture having a suture needle on at least one suture deployment end. "Suture deployment end" refers to an end of the suture to be deployed into tissue; one or both ends of the suture may be suture deployment ends. The suture deployment end may be attached to a deployment device such as a suture needle, or may be sufficiently sharp and rigid to penetrate tissue on its own.

"Wound closure" refers to a surgical procedure for closing of a wound. An injury, especially one in which the skin or another external or internal surface is cut, torn, pierced, or otherwise broken is known as a wound. A wound commonly occurs when the integrity of any tissue is compromised (e.g., skin breaks or burns, muscle tears, or bone fractures). A wound may be caused by an act, such as a puncture, fall, or surgical procedure; by an infectious disease; or by an underlying medical condition. Surgical wound closure facilitates the biological event of healing by joining, or closely approximating, the edges of those wounds where the tissue has been torn, cut, or otherwise separated. Surgical wound closure directly apposes or approximates the tissue layers, which serves to minimize the volume new tissue formation required to bridge the gap between the two edges of the wound. Closure can serve both functional and aesthetic purposes. These purposes include elimination of dead space by approximating the subcutaneous tissues, minimization of scar formation by careful epidermal alignment, and avoidance of a depressed scar by precise eversion of skin edges.

'Tissue elevation procedure" refers to a surgical procedure for repositioning tissue from a lower elevation to a higher elevation (i.e. moving the tissue in a direction opposite to the direction of gravity). The retaining ligaments of the face support facial soft tissue in the normal anatomic position. However, with age, gravitational effects and loss of tissue volume effect downward migration of tissue, and fat descends into the plane between the superficial and deep facial fascia, thus causing facial tissue to sag. Face-lift procedures are designed to lift these sagging tissues, and are one example of a more general class of medical procedure known as a tissue elevation procedure. More generally, a tissue elevation procedure reverses the appearance change that results from effects of aging and gravity over time, and other temporal effects that cause tissue to sag, such as genetic effects. It should be noted that tissue can also be repositioned without elevation; in some procedures tissues are repositioned laterally (away from the midline), medially (towards the midline) or inferiorly (lowered) in order to restore symmetry (i.e. repositioned such that the left and right sides of the body "match").

"Medical device" or "implant" refers to any object placed in the body for the purpose of restoring physiological function, reducing/alleviating symptoms associated with disease, and/or repairing and/or replacing damaged or diseased organs and tissues. While normally composed of biologically compatible synthetic materials (e.g., medical-grade stainless steel, titanium and other metals or polymers such as polyurethane, silicon, PLA, PLGA and other materials) that are exogenous, some medical devices and implants include materials derived from animals (e.g., "xenografts" such as whole animal organs; animal tissues such as heart valves; naturally occurring or chemically-modified molecules such as collagen, hyaluronic acid, proteins, carbohydrates and others), human donors (e.g., "allografts" such as whole organs; tissues such as bone grafts, skin grafts and others), or from the patients themselves (e.g., "autografts" such as saphenous vein grafts, skin grafts, tendon/ligament/muscle transplants). Medical devices that can be used in procedures in conjunction with the present invention include, but are not restricted to, orthopedic implants (artificial joints, ligaments and tendons; screws, plates, and other implantable hardware), dental implants, intravascular implants (arterial and venous vascular bypass grafts, hemodialysis access grafts; both autologous and synthetic), skin grafts (autologous, synthetic), tubes, drains, implantable tissue bulking agents, pumps, shunts, sealants, surgical meshes (e.g., hernia repair meshes, tissue scaffolds), fistula treatments, spinal implants (e.g., artificial intervertebral discs, spinal fusion devices, etc.) and the like.

### MARKED HETEROFUNCTIONAL SUTURES

As discussed above, the present disclosure provides compositions, configurations, methods of manufacturing and methods of using sutures, heterofunctional sutures and self-retaining sutures in surgical procedures which eliminate a variety of limitations and provide enhanced and/or additional functionality. As used herein, a heterofunctional suture is a suture having two or more functionally distinct sections of suture filament where the sections of filament have different features. A heterofunctional suture may also encompass sutures having two or more sections of filament where devices associated with the sections of filament, such as a needle by way of example only, have different features. As used herein the term "feature" is used to refer to a fixed property of a suture, such as material, retainer orientation, nominal diameter, needle configuration etc. The term "condition" is used to refer to variable properties of a suture filament such as tension, temperature etc. The term property is used to encompass both features (fixed properties) and conditions (variable properties) of sutures. The two or more sections of suture filament in a heterofunctional suture need not be of any particular length, but a section should be long enough for its difference in property to have an effect on the functionality of the section. This typically requires a length of suture long enough for at least one pass or bite through tissue under the conditions of use. Typically "a section of suture" will be a portion of suture having a length at least two orders of magnitude larger than the diameter and more typically, three or four orders of magnitude larger than the diameter of the suture.

In accordance with particular embodiments, the present invention provides self-retaining sutures which are dual-armed sutures; triple- armed sutures; multiple-armed sutures; heterofunctional sutures having two or more sections of suture having different features; dual-arm sutures having different types (or sizes) of needles on each end; single or dual-armed sutures for use with different layers/depth and types of tissue; single or dual armed sutures with sections of filament having different diameters for use with different layers/depth and types of tissue; dual-armed sutures having asymmetrically placed transition sections; and sutures having a combination of two or more of these features. According to particular embodiments of the present invention, sections of self-retaining sutures may be unmarked, marked or differentially-marked by one or more types of markers or combination of markers. Marked sutures include by way of example, dual-armed sutures having different markers on each end of the suture; heterofunctional sutures having different markers on different sections of suture; self-retaining sutures having markers indicative of the presence, absence and/or orientation of retainers in a section of suture; dual-armed sutures having different markers on each needle; dual-armed sutures having markers to identify orientation or direction of an end of a dual armed suture; sutures having markers utilized in robotically assisted surgical tools and with endoscopic surgical tools; sutures having markers that identify types and characteristics of sutures and call up such data from tables in computer devices (computer-assisted surgery devices) which display that information for the doctor or limit what the doctor can do as far as tensioning the suture as deployed; and sutures having markers in combination with sound or variable sound generators or light or variable light generators or haptic devices that vary a stimulus provided to a physician depending on the stress, strain and/or tension on the sutures.

In addition to marked sutures for facilitating the doctor's use of the suture during a surgical procedure, sutures disclosed herein include ones with markers that can be used for identifying sections of a heterofunctional suture after it is has been deployed and the surgical procedure completed. For example, markers detectable by computed tomography or computed axial tomography can assist in the locating of suture well after the patient has healed from the surgical procedure.

The markers may be provided on the suture or on a needle or on another device associated with a suture or section of suture for example a pledget or the like. The markers include, but are not limited to: markers which identify features of the suture such as materials and/or other fixed properties; markers which identify conditions of the suture such as tension and/or other variable properties; markers visible in the visible light frequency range, markers visible to the naked eye but which are visualized under the conditions of surgical use; markers recognizable in the non-visible radiation frequency range, such as in the ultraviolet or other fluorescence spectra; markers detectable with ultrasound; markers detectable by x-ray radiation; markers detectable by magnetic resonance imaging; markers detectable by computed tomography (CT) scans or by computed axial tomography (CAT) scans; markers which are machine readable; markers which may be read remotely; markers which are active markers; markers which are passive markers (passive RFID); markers which include an LED and an accelerometer or strain sensor; markers which include a light source and a sensor responsive to conditions of the suture; markers which identify the presence, absence and/or orientation of retainers; markers which identify different sections of a suture having different features; markers which change color due to suture stress, strain and/or tension; markers with alternating colors where the colors blend and produce a different color in response to suture stress, strain and/or tension; strain and/or tension; markers with different colors placed at different depths or side by side where the colors blend and produce a different color in response to suture stress, strain and/or tension; markers having one or more patterns where the patterns interfere with each to produce a visible or recognizable change in pattern in response to suture stress, strain and/or tension; markers which span a stretchable suture body and a relatively not stretchable retainer (somewhat isolated from the body) such that there is a noticeable misalignment when the suture body is under stress and stretched; and markers which deform or change configuration when the suture is under stress; markers which extend from housings or sleeves or cavities in the needle or suture when the suture is under stress; markers useful with stereo/3D imaging devices.

### A. Marked Heterofunctional Self-Retaining Suture Systems

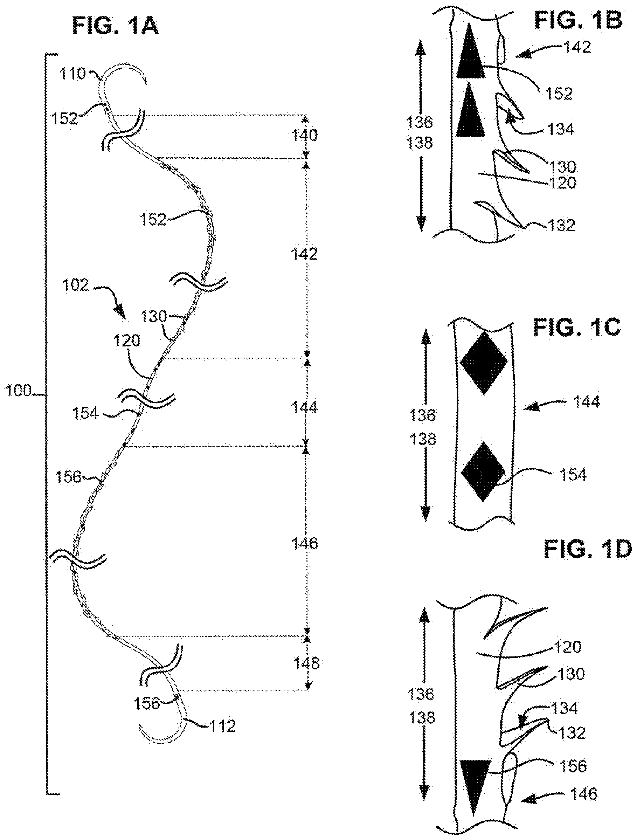

FIG. 1A illustrates a heterofunctional self-retaining suture system 100. Self-retaining suture system 100 comprises needles 110, 112 attached to self-retaining suture thread 102. Self-retaining suture thread 102 includes a plurality of retainers 130 distributed on the surface of a filament 120. In lead-in section 140 of filament 120 there are no retainers 130. In section 142 of filament 120 there are a plurality of retainers 130 arranged such that the suture can be deployed in the direction of needle 110, but resists movement in the direction of needle 112. In transition section 144, there are no retainers 130. In section 146, there are a plurality of retainers 130 arranged such that the suture can be deployed in the direction of needle 112, but resists movement in the direction of needle 110. The retainers 130 in section 146 are larger than the retainers 130 in section 142. The larger retainers are better suited for gripping tissue that is softer and/or less dense than the smaller retainers. In lead-in section 148 of filament 120 there are no retainers 130.

A break is shown in each of sections 140, 142, 144, 146 and 148 to indicate that the length of each section may be varied and selected depending upon the application for which the suture is intended to be used. For example, transition section 144 can be asymmetrically located closer to needle 110 or needle 112, if desired. A self-retaining suture having an asymmetrically located transition section 144 may be favored by a physician that prefers to use his dominant hand in techniques that require suturing in opposite directions along a wound. The physician may start further from one end of the wound than the other and stitch the longer portion of the wound with the needle that is located further from the transition section 144. This allows a physician to use his dominant hand to stitch the majority of the wound with the longer arm of the suture. The longer arm of the suture is that section of suture between the transition section and the needle which is located further from the transition section.

Heterofunctional self-retaining suture system 100 is composed of two arms having different functions. Each arm may be considered to be a section of self-retaining suture system 100. The first arm comprising sections 142 and section 140 of self-retaining suture thread 102 and a curved needle 110 has relatively small retainer suitable for engaging harder/denser tissue. The second arm comprising sections 146 and 148 and needle 112 of self-retaining suture thread 102 has relatively larger retainers suitable for engaging softer/less dense tissue. Self-retaining suture thread 102 of FIG. 1A is a heterofunctional suture thread because each arm of the suture has different features. For example, as previously stated, the first arm has relatively small retainer suitable for engaging harder/denser tissue and the second arm has relatively larger retainers suitable for engaging softer/less dense tissue. During surgery, it may however, be difficult for the physician to differentiate one arm or needle of self-retaining suture system 100 from the other arm or to identify and differentiate one arm or needle of self-retaining suture thread 102 the other arm. Thus, in accordance with certain embodiments of the present invention, self-retaining suture system 100 can be provided with visible markers associated with one or other of the arms or needle of the self-retaining suture system or sections of the heterofunctional suture thread 102. The visible markers enable a physician to recognize and differentiate the arms of the heterofunctional self-retaining suture system 100 and/or the sections of the heterofunctional suture thread 102.

FIG. 1B illustrates a magnified view of self-retaining suture thread 102 in section 142. As shown in FIG. 1B, a plurality of retainers 130 is distributed on the surface of filament 120. The affixation of self-retaining sutures after deployment in tissue entails the penetration of retainer ends 132 into the surrounding tissue resulting in tissue being caught between the retainer 130 and the body of suture filament 120. The inner surface 134 of the retainer 130 that is in contact with the tissue that is caught between the retainer 130 and the body of filament 120, is referred to herein as the "tissue engagement surface" or "inner retainer surface." As illustrated in FIG. 1B, each retainer 130 has a tip 132 and tissue retainer surface 134. When self-retaining suture thread 102 is moved in the direction of arrow 136, retainers 130 lies flat against the body of filament 120. However, when self-retaining suture thread 102 is moved in the direction of arrow 138, tip 132 of retainer 130 engages tissue surrounding filament 120 and causes retainer 130 to fan out from filament 120 and engage the tissue with tissue engagement surface 134 thereby preventing movement of the suture in that direction. As shown, in FIG. 1B, the filament 120 in section 142 carries a marker 152 composed of two triangles. The tip of the triangles point in one direction along the longitudinal axis of the filament 120. This direction shown by arrow 136 is the direction in which section 142 of self-retaining suture thread 102 can be deployed without retainers 130 engaging tissue. Thus marker 152 both identifies section 142 and indicates its orientation. As shown in FIG. 1A, needle 110 may also be marked with the same marker 152.

FIG. 1C illustrates a magnified view of self-retaining suture thread 102 in section 144. As shown in FIG. 1C, in section 144, there are no retainers 130. Section 144 may be referred to as the transition section of self-retaining suture system 100. Section 144 may be deployed in either both of the directions shown by arrows 136 and 138. In many procedures it is desirable to locate the transition region in order to properly situate the transition region at the beginning of suture deployment. As shown, in FIG. 1C, the filament 120 in section 144 carries a marker 154 composed of a diamond. The tips of the diamonds point in both directions, illustrating that section 144 of self-retaining suture thread 102 can be deployed in either direction and differentiating section 144 from section 142.

FIG. 1D illustrates a magnified view of self-retaining suture thread 102 in section 146. As shown in FIG. 1D, a plurality of retainers 130 is distributed on the surface of filament 120. As illustrated in FIG. 1D, each retainer 130 has a tip 132 and tissue retainer surface 134. When self-retaining suture thread 102 is moved in the direction of arrow 138, retainer 130 lies flat against the body of filament 120. However, when self-retaining suture thread 102 is moved in the direction of arrow 136, tip 132 or retainer 130 engages tissue surrounding filament 120 and causes retainer 130 to fan out from filament 120 and engage the tissue with face 134, thereby preventing movement of the suture in that direction. Thus, in section 146 retainers 130 are oriented in the opposite direction to the retainers 130 in section 142. As shown, in FIG. 1D, the filament 120 in section 146 carries a marker 156 composed of a single triangle. The tip of the triangle points in the direction of arrow 138 in which section 146 of self-retaining suture thread 102 can be deployed without retainers 130 engaging tissue. Note that marker 156 is different than marker 152 allowing the arms of self-retaining suture system 100 to be recognized and differentiated. Thus marker 156 identifies the orientation of the retainers in section 146 and differentiates section 146 from sections 144 and 142. As shown in FIG. 1A, needle 112 may also be marked with the same marker 156. Additionally, marker differences can include different shapes, different colors, different numbers, and different letters to name a few types of markers.

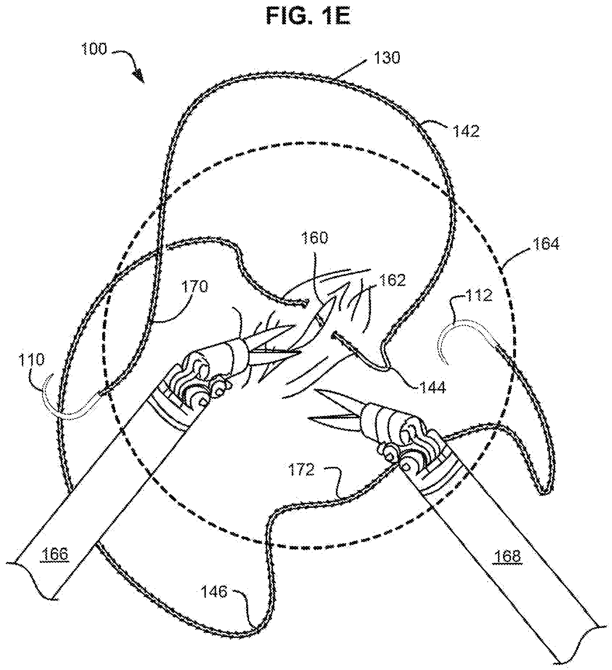

FIG. 1E illustrates the problem solved by the present invention. As shown in FIG. 1E, an unmarked self-retaining suture system 100 is used in a cavity of the human body to close an opening 160 in tissue 162. The procedure is being performed endoscopically and the visual field 164 of the physician is limited to the section inside the dashed circle. The physician has taken a bite through the tissue 162 on both sides of opening 160. Two endoscopic instruments 166 and 168 are controlled by the physician and are visible to the physician within the operative field 164. The physician has taken a bite through the tissue 162 on each side of opening 160 with needle 112 and drawn section 146 through the tissue to the position shown in FIG. 1E. The physician has temporarily released self-retaining suture system 100 with the endoscopic instruments. Needle 112 and a portion of self-retaining suture system 100 are outside of the visual field.

The physician wishes to move the transition section 144 so that it is approximately centered upon opening 160 and then pick up needle 112 and take another bite through the tissue on each side of opening 160 moving from right to left. However, the retainers 130 along are not sufficiently visible to the physician via the endoscope. Also, endoscopic instruments 166 and 168 do not provide enough tactile sensation to the physician for the physician to be able to feel where the retainers 130 are located.

The first task for the physician is how to identify section 144, differentiate it from sections 142 and 146 and then center section 144 upon opening 160. If section 144 is provided with markers 154, as shown in FIGS. 1A, 1C the physician can identify section 144, differentiate it from sections 142 and 146 and then pull the suture through until section 144 is centered upon opening 160.

The next task for the physician is finding and identifying the needle associated with the suture exiting on the right side of the opening 160. Note that section 142 of the suture which is located on the right of opening 160 leaves visual field 164. Everything outside the dashed circle is invisible to the physician without moving the endoscope. Unless needle 112 is marked in some way, the physician may assume, incorrectly that needle 112 is associated with section 142 of self-retaining suture system 100. However, if needle 112 is marked as shown in FIG. 1A, then the physician can identify needle 112 as the incorrect needle.

The next task for the physician is to find and grasp needle 110. One way for the physician to acquire needle 110 is to follow section 142 of the suture all the way from opening 160 to the end. This is time consuming and the physician maybe come confused if sections 142 and 146 cross or move at some point. A faster technique would be for the physician to start from the visible segment 170 of section 142 of the suture within the visual field 164. However, unless section 142 of the suture thread is marked in some way there is no way for the physician to be sure that visible segment 170 of the suture is part of section 142. Likewise the physician cannot tell whether visible segment 172 of the suture is part of section 142 or part of section 146. If section 142 is marked in some way, the physician may acquire section 142 at visible portion 170. If section 142 is marked in a way that indicates orientation of the suture the physician will be able also to know in what direction needle 110 lies from the visible portion 170 allowing the physician to acquire the needle 110 in the most expedient and accurate way. If needle 110 is also marked in some way, the physician may confirm that the physician has acquired the correct needle for the next step in the procedure. Thus, marking the suture, suture sections and/or needles reduces error and saves time.

### B. Heterofunctional Self-Retaining Suture Systems

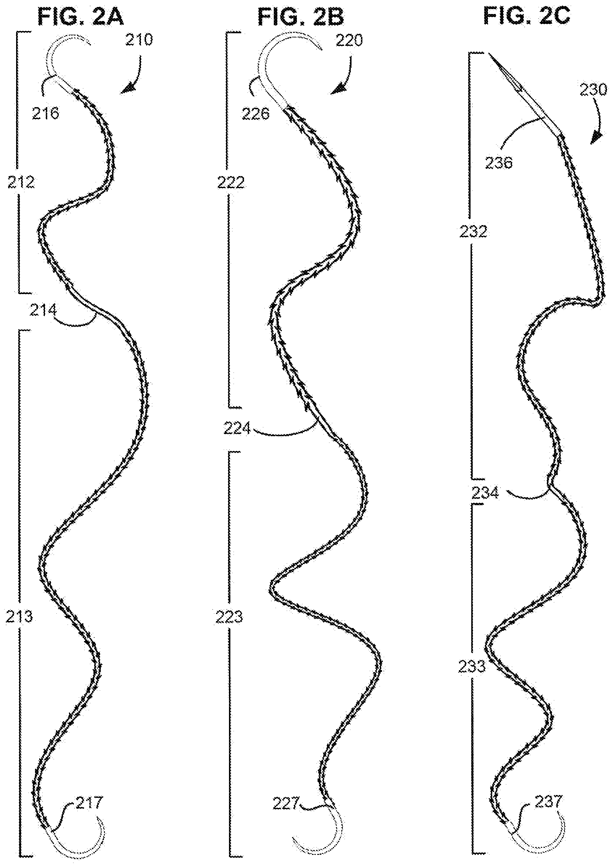

As discussed above, it is particularly desirable to mark and identify portions of a self-retaining system when there is a difference in the features/utility of different sections of the self-retaining suture. In the case of self-retaining sutures the difference in features between sections of the suture may be the presence, absence and orientation of retainers associated with the section. In FIG. 1A, the difference in function between the two arms of self-retaining suture system 100 is the length of retainers 130. However, the differences in features and/or utility between sections of a heterofunctional suture are not limited to the presence, absence and orientation of retainers and include such differences as needle length, needle diameter, needle configuration, needle tip configuration; needle attachment method, needle material, needle surface treatment, suture length, suture diameter, suture material, suture manufacturing process, suture coating, suture texture, suture surface treatment, associated pharmaceuticals, suture shape-memory features, suture strength, suture elasticity, suture hardness, suture absorbability, retainer configuration, retainer dimensions, retainer distribution, and retainer elevation. FIGS. 2A - 2D show examples of heterofunctional sutures having two or more sections having different features according to embodiments of the present invention.

As shown in FIG. 2A, an example of a heterofunctional self-retaining suture system 210 includes two arms 212, 213 joined at a transition section 214. Each arm 212, 213, terminates in a needle 216, 217. The length of suture filament in arm 212 however is significantly shorter than the length of suture filament in arm 213. This heterofunctional self-retaining suture system is useful for example in a wound closure where a physician begins at the center point of the wound and the sutures towards one end of the wound with one arm of the suture and towards the other end of the wound with the other arm of the suture. Usually a physician is more comfortable suturing in one direction rather than the other. Thus with the heterofunctional self-retaining suture system 210 of FIG. 2A, the physician may commence closer to one end of the wound instead of in the center of the wound. Thus the physician can perform more suturing with arm 213 in the preferred direction and less suturing with arm 212 in the less preferred direction.

As shown in FIG. 2B, another example of a heterofunctional self-retaining suture system 220 includes two arms 222, 223 joined at a transition section 224. Each arm 222, 223, terminates in a needle 226, 227. The suture filament in arm 222 is, in this embodiment, significantly thicker than the thickness of the suture filament in arm 223. In addition needle 226 of arm 222 is correspondingly larger than needle 227 of arm 223. This heterofunctional self-retaining suture system is useful for example in a wound closure where a physician uses one arm of the suture to close deeper tissue and uses another arm of the suture to close superficial tissue. Typically, it is preferable to use a finer suture for the superficial closure to reduce tissue reactivity to the suture and provide enhanced cosmesis. With the heterofunctional self-retaining suture system of FIG. 2B, the physician may first utilize arm 222 for the deep wound closure, where the need for strength is a more significant factor, and may then use arm 223 for closing the surface of the wound, where the need for reduced tissue reactivity and enhanced cosmesis are more significant factors.

The difference in function between the arms in a bidirectional self-retaining suture system may be due to differences in the needles (or other devices attached to the filament) rather than the suture filament itself. For example, as shown in FIG. 2C, another exemplary heterofunctional self-retaining suture system 230 includes two arms 232, 233 joined at a transition section 234. Each arm 232, 233, terminates in a needle 236, 237. The suture filament in arm 232 is the same thickness and length as the suture filament 233 and has a similar retainer configuration (retainers in each arm oriented to allow deployment in direction of the needle of that arm and resist movement in the opposite direction). However, arm 232 includes a straight needle 236 whereas arm 233 includes a curved needle 237. Different surgical needles are used by physicians for different suturing, tissue approximation and tissue elevation techniques thus it is useful to have facility to use different techniques provided in one self-retaining suture. With the heterofunctional self-retaining suture system 230 of FIG. 2C, the physician may utilize straight needle 236 of arm 232 using one technique and utilize curved needle 237 of arm 233 using a different technique.

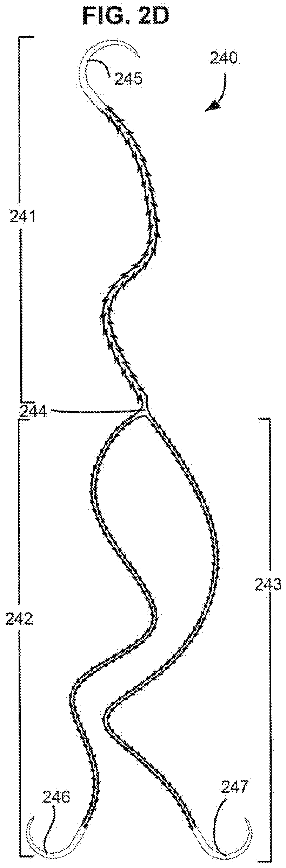

Heterofunctional self-retaining suture systems are not limited to two arms (dual-armed suture). A heterofunctional self-retaining suture system may have more than two arms. Other multiple-arm sutures may include two, three, four, five or more arms. As shown in FIG. 2D, another example of a heterofunctional self-retaining suture system 240 includes three arms 241, 242, 243 joined at a transition section 244. Each arm 241, 242, 243 terminates in a needle 245, 246, 247. Arms 242 and 243 have the same type of filament and needle. The suture filament in arm 241 is however significantly thicker than the thickness of the suture filament in arms, 242 243. In addition needle 245 of arm 241 is correspondingly larger than needles 246, 247 of arms 242, 243. This heterofunctional self-retaining suture system is useful for example in a wound closure where a physician uses one arm of the suture to close deeper tissue and uses another arm of the suture to close superficial tissue. Typically, it is preferable to use a finer suture for the superficial closure to reduce tissue reactivity to the suture and provide enhanced cosmesis. With the heterofunctional self-retaining suture system of FIG. 2B, the physician may first utilize arm 241 for the deep wound closure, where the need for strength is more significant, and may then use arms 242, 243 for closing the surface of the wound, where tissue reactivity and cosmesis are more significant.

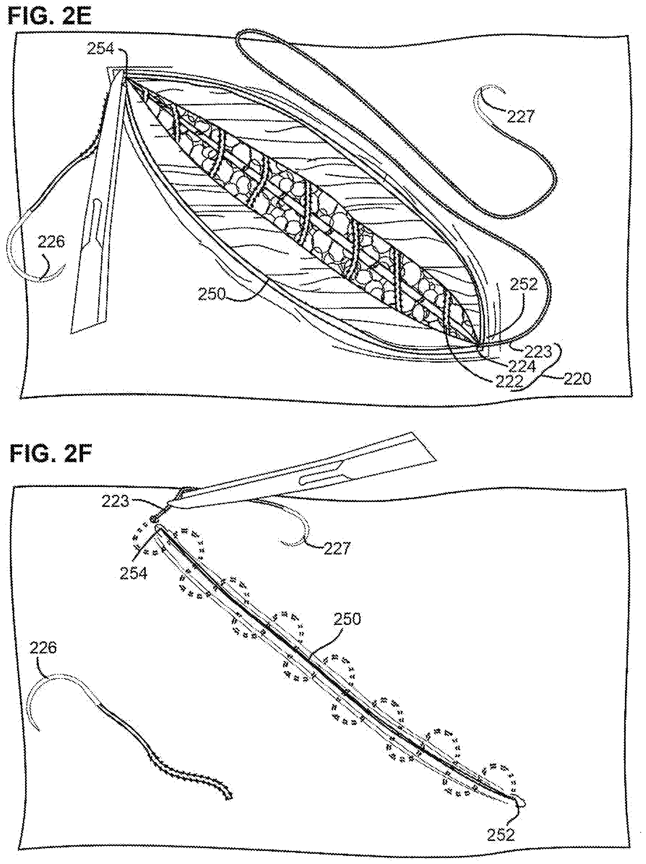

FIGS. 2E and 2F show an example of a multiple-layer wound closure using a heterofunctional suture such as shown in FIG. 2B. As shown in FIG. 2B, heterofunctional self-retaining suture system 220 includes two arms 222, 223 joined at a transition section 224. Each arm 222, 223, terminates in a needle 226, 227. The suture filament in arm 222 is significantly thicker than the thickness of the suture filament in arm 223. In addition needle 226 of arm 222 is correspondingly larger than needle 227 of arm 223. As shown in FIGS. 2E and 2F, the physician closes the wound 250 beginning at a first end 252 and suturing towards the second end 254. The physician closes wound 250 in two layers. The physician first utilizes arm 222 for the deep wound closure (shown in FIG. 2E) and then uses arm 223 for closing the surface of the wound (shown in FIG. 2F). One advantage of this closure technique is that the surgeon can select which end (252 or 254) of the wound 250 to commence the closure and can the close the wound suturing in one direction, thus the physician can suture exclusively with their dominant hand and avoid changing from one side of the patient to the other during suturing.

Referring now to FIG. 2E which shows closure of the deep tissue layer. The physician passes needle 226 through the edge of the subcuticular layer, deep dermis and the soft tissue and draws arm 222 through the wound until the retainers of arm 223 begin to engage the tissue. The physician closes the deep layers using a helical or sinusoidal stitch configuration. Because needle 226 is relatively larger, the physician can take large bites through the tissue in this deep layer closure which allows a greater tension force to be applied to the tissue to close the wound without tearing the tissue or causing ischemia. The surgeon may tension arm 222 as the suturing progresses from end 252 to end 254 thereby progressively closing the wound 250. If necessary, the physician may make a subcutaneous pass with needle 227 to set more retainers of arm 223 in tissue to prevent pull through while closing the deep layers of tissue with arm 222. The suture filament of arm 222 is relatively thicker and thus allows for application of greater tension to close the wound 250 than would otherwise have been possible.

Referring now to FIG. 2F which shows the superficial portion of the wound closure. As shown in FIG. 2F, the physician passes through the subcutaneous tissue taking bites on alternate sides of the wound. The depth of the needle as it enters the tissue and emerges should be the same for each bite and on the opposite side of the wound. The radius of the bite is determined by the radius of needle 227. The bites are thus smaller than the bites made with arm 222 and needle 226. Thus less tension can be applied to the wound 250 by arm 223. However, the majority of the tension required to close wound 250 has already been applied by arm 222. The smaller diameter filament and smaller bites of needle 227 are therefore sufficient to close the superficial wound as shown in FIG. 2F. Additionally, because arm 223 is made of a relatively smaller diameter filament, there will be less tissue reactivity to the suture filament. This will enhance cosmesis and reduce adverse results such as splitting and the like. As the wound closure progresses the physician may wish to apply further tension to the deep layer suture arm 222. When suitable tension has been achieved the physician may make a J- loop pass with the suture and cut off the remaining suture and needle of arm 222. When the physician finished the superficial closure, the physician takes the last subcutaneous bite through tissue 2cm beyond the second end 254 of the wound 250 exiting through the skin. The physician pushes down on the tissue and cuts of the remaining needle and suture flush with the skin.

### C. Visible Suture Markers For Heterofunctional Sutures

As discussed above, it is particularly desirable to mark and identify portions of a heterofunctional suture system where different sections of the suture have different features such as in dual-arm self-retaining suture systems. In heterofunctional self-retaining suture systems the difference in function between sections of the suture may be the presence, absence and/or orientation of retainers. To serve the purpose of allowing a physician to identify and differentiate suture sections, the suture markers should be readily recognized and distinguished by the physician under the conditions in which the suture is to be used. For example, in microsurgery applications, markers may be used that are visible under the microscope, but not necessarily visible to the naked eye. Likewise in endoscopic applications, markers should be used that are visible through the endoscope and associated display system. If the suture will be used with fluoroscopic visualization then the markers may include radiopaque markers. If the suture will be used with ultrasound visualization then the markers may include echogenic markers. Thus, different markers and different types of markers may be appropriate under different circumstances depending upon the circumstances of the procedure and the scanning/imaging/visualization technology utilized in the procedure.

The markers can be provided in various forms that may be identified and distinguished from one another. The markers may comprise distinguishable, patterns, shapes, lengths, colors sizes, directions and arrangements. The markers can include different colors such as red, green, orange, yellow, green, blue etc. Such colors may be used in a uniform density or varying density in which case the graduation of color density may be used to designate e.g. an orientation. The markers may be included along the entire length of the self-retaining suture system, at a number of discrete points, or only at the ends or transition section of the self-retaining suture. In some cases it may be desirable to use a color for markers that is uncommon in the operative environment. For example, it may be desirable to use green markers because green is not common in the human body. In endoscopic applications using green is advantageous because the video system can be programmed to emphasize green and enhance marker visualization without interfering with the remainder of the image.

The markers can be formed by various conventional methods. For example, the markers can be coated, sprayed, glued, dyed, stained, or otherwise affixed to the self-retaining suture systems or components thereof. Traditional colourant application processes include, without limitation, dipping, spraying (by, for example, an ink jet), painting, printing, applying and/or coating colourants on the suture section of interest. Critical fluid extraction (such as carbon oxide) may also be used to add colourant locally to all or part of the section desired to be marked. Alternatively, colourant(s) for the suture section of interest may be included in a portion of the suture material that is used to form the suture body, wherein that portion is in the section of interest of the manufactured suture.

Additionally, the suture section of interest can be demarcated by using an energy-activated colourant. For example, when a laser-activated colourant (that is, a pigment or dye which permanently changes colour after being exposed to laser energy) is used to colour the suture, then the suture section of interest can be demarcated by using laser energy to permanently change the suture coating in the suture section of interest. This also applies to using other energy activated colourants which are activated by other energy sources such as, but not limited to, heat, chemicals, microwaves, ultraviolet light, or x-rays. For example, bleaching chemicals such as sodium hypochlorite or hydrogen peroxide will permanently change the colourant's colour which allows for the demarcation of the suture section of interest.The marking of the suture section of interest may be one or more mechanical tabs. This tab can be made from both absorbable or non-absorbable materials. For example, a non-absorb tab made from polyester felt or polytetrafluoroethylene felt can be used as the transition marker were the surgeon gently pulls and removes the tab from the suture once the suture section of interest is position in the tissue prior to completing the tissue closure of the wound. The removal of the tab can be facilitated by having a cut or slice partially through the tab for ease of removal during surgery. Examples of absorbable materials include glycolide and glycolide-lactide polymers. The configuration of the tab includes, but not limited to, a rectangle tab, a circular tab, or a spiral tab. For example, but not limited to, the tab can be affixed to the suture with a hole were the suture passes through the tab, a partial cut/slice where the suture is positioned in the tab slice, by folding the table around the suture, by crimping the tab to the suture, by twisting the tab around the suture, by tying the tab to the suture, or by adhesion (e.g., ultrasonic welding, adhesive "gluing", thermal welding, and the like) to the suture, The use of an absorbable tab can be especially useful for deep cavity tissue closures where the surgeon may choose to leave the absorbable tab inside the body.

Additionally, the mechanical tab can be coloured to improve the visibility of the tab to mark the suture section of interest. This includes, but not limited to, using fluorescent colourants, radio detectable compounds, or magnetic resonance imaging detectable compounds.

Additionally, the colourant(s) employed for demarcating the suture section of interest may be included on a plastic biocompatible material which is applied on the suture at the section of interest. Such a layer may be absorbable, such as polyglycolide coating which has a colourant to mark the suture section of interest, or it may be a non-absorbable material, such silicone. The coloured material may be synthetic or may be derived from a natural source (whether the material be modified or unmodified), such as collagen. The plastic biocompatible material may be applied to the suture before or after the retainers are formed on the suture body.

Alternatively, the suture section of interest may be reverse-marked, such that where the suture body is already visibly coloured, the colourant may be absent from all or part of the suture section of interest such that at least a portion of the section of interest is optically distinguishable by the surgeon from the rest of the suture. Such a suture may manufactured by including a colourant-free portion of suture material in the suture section of interest area during the manufacture of the suture body (for example, by extrusion) or by removal of colourant from the suture section of interest after the suture body has been manufactured, whether before or after retainers have been formed on the suture body. Colourant may be removed locally by, for example, critical fluid extraction such as (e.g., carbon oxide). It is not necessary to remove all of the colourant from the section of interest of the suture as long as there is a difference detectible by a surgeon between the section of interest and the rest of the suture.

Another example of a reverse-marked suture is one that lacks a coloured layer that is present on the rest of the suture body. A plastic biocompatible material bearing a colourant may be applied on the other sections of the suture, and at least where the other sections border the section of interest. Examples of such materials are discussed above. As in the foregoing examples, demarcating the suture section of interest may be effected in the suture manufacturing process either before or after forming retainers.

Another example of a reverse-marked suture is one having a coaxial structure wherein each coaxial layer having a different colour, and a portion of the outermost layer(s) is removed to visually expose a layer below. For example, a dual-layer monofilament polypropylene suture can be produced with a white inner core (intercoaxial layer) with a blue outer coaxial layer, and portions of the outer layer can be removed to visually expose the white inner monofilament to mark the suture section of interest.

Yet another example of a reverse-marked suture is one in which an external coating is removed (or partially removed) from the suture in the suture section of interest, and where either the coating or base suture has a contrasting colour difference. This technique of removing (or partially removing) material in the suture section of interest may also create a tactile demarcation of the suture section of interest.

The marking may include a radio-detectable compound or magnetic resonance imaging detectable compound. For example a suture section of interest provided with barium sulfate (BaSO₄), such as by impregnating the suture with barium sulfate or adding a coating containing barium sulfate, will be detectable by electromagnetic energy. In the case of x-ray detection, the barium sulfate marked section of interest would be radiopaque. Likewise, computed tomography (CT) scans or computed axial tomography (CAT) scans can be used to detect the radio detectable section of interest. The use of electromagnetic energy for radio detection of the transition section is not limited to using x-ray wavelengths as other radio frequencies may be used. Likewise, gadolinium (Gd) or gadolinium compounds can be used for the marking of the suture section of interest especially when the detection will be done by using magnetic resonance imaging (MRI). The use of radio detectable or magnetic resonance imaging detectable marking of the transition zone may be useful to the surgeon during laparoscopic surgical procedures.

Alternatively, markers can be made by treating a surface of the suture system to make an visually observable change in surface characteristics such as by branding, texturing, embossing, stamping and the like. Alternatively, the markers may be an integral part of the material from which the self-retaining suture system is formed - such as by forming a self-retaining suture system by joining different sections of suture filament of different colors. The markers may be provided on one or more of the suture filament, the needles, or another item, such as a pledget, associated with the self-retaining suture system or section of the filament. In some case markers may be formed as an integral part of the retainers of a self-retaining suture, such as by creating retainers of a particular color of material or by exposing a particular color of material - different retainer patterns may be used to differentiate different sections of the self-retaining suture.

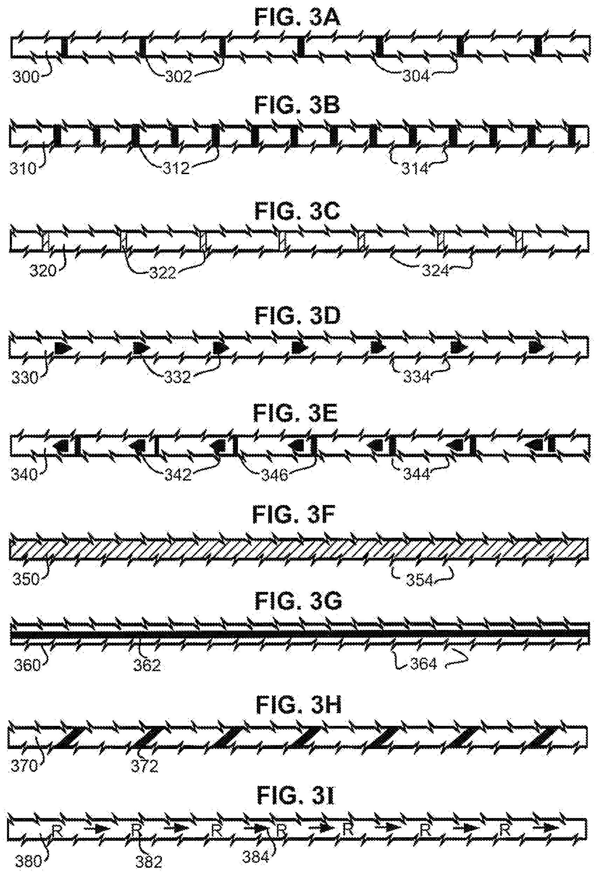

FIGS. 3A-3I illustrate some of the many ways in which markers on the suture filament may be used to identify one section of suture and distinguish it from other sections. The markers may be used alone or in combination with other of the markers. The markers may identify, differentiate and/or delineate sutures and/or sections of suture having different features.

FIG. 3A shows a section 300 of a self-retaining suture filament. The section 300 is marked at regular intervals with bands 302 of a solid color distinguishable from the color of the filament. The bands serve to identify section 300 but, standing alone, do not indicate the orientation of the retainers 304 within the section 300.

FIG. 3B shows a section 310 of a self-retaining suture filament. The section 310 is marked at regular intervals with bands 312 of a solid color distinguishable from the background color of the filament. The bands serve to identify section 310 but, standing alone, do not indicate the orientation of the retainers 314 within the section 300. The interval between bands 312 of section 310 is significantly less than the interval between the bands 302 of section 300. The different arrangement of bands 312 and bands 302 may be visually observed and thus allows the physician to distinguish section 310 from section 300.

FIG. 3C shows a section 320 of a self-retaining suture filament. The section 320 is marked at regular intervals with bands 322 of a solid color distinguishable from the background color of the filament. The bands serve to identify section 320 but, standing alone, do not indicate the orientation of the retainers 324 within the section 320. The color or color density of bands 322 of section 320 is distinguishable from the color (or color density) of bands 302 of section 300 (represented by shading in FIG. 3C by the shading of bands 322). The different color and/or color density of bands 322 and bands 302 may be visually observed and thus allows the physician to distinguish section 320 from section 300.

FIG. 3D shows a section 330 of a self-retaining suture filament. The section 330 is marked at regular intervals with shapes 332 of a solid color distinguishable from the background color of the filament. The shapes have a discernible orientation which is asymmetric relative to the longitudinal axis of section 330. For example shapes 332, can be regarded as pointing in the direction of the apex on the right side. Thus shapes 332 can be used to identify section 330 and also to indicate the orientation of the retainers 304 within the section 300. The direction in which the markers point can be assigned by convention, however, in the section 330, shapes 332 point in the direction in which the section 330 of self-retaining suture filament may be deployed through tissue without resistance by retainers 334. As part of a self-retaining suture system the shapes would thus point along the filament in the direction of the deployment needle associated with the arm including section 330. The different arrangement of bands 332 and bands 302 may be visually observed and thus also allows the physician also to distinguish section 330 from section 300.

The shapes of FIG. 3D may be used to indicate the orientation of a self-retaining suture filament. FIG. 3E shows a section 340 of a self-retaining suture filament oriented in the opposite direction to section 300. The section 340 is also marked at regular intervals with shapes 342 of a solid color distinguishable from the background color of the filament. Shapes 342, can be regarded as pointing in the direction of the apex on the left side. However, without more section 340 could be confused with section 330. Thus an additional feature is used to differentiate section 340 from section 330. As shown in FIG. 3D, section 340 is marked at regular intervals with bands 346 of a solid color distinguishable from the color of the filament in addition to shapes 342. Thus section 340 can be identified and distinguished from section 330 and the orientation of the filament in each of sections 330, 340 may be determined from the markers.

FIG. 3F shows a section 350 of a self-retaining suture filament. The section 350 is formed of a material having a solid color or marked with a solid color that is distinguishable from other sections of the suture. The color serves to identify section 350 but, standing alone, does not indicate the orientation of the retainers 354 within the section 350. The color may be visually observed and thus allows the physician to distinguish section 350 from a section of a different color. Different colors may be used to identify sections of a self-retaining suture system either by joining different colored sections of suture after they have been colored or by treating different sections of the same filament to have different colors such as dying a section of a filament to add color, or treating a section of a filament to remove color.

FIG. 3G shows a section 360 of a self-retaining suture filament. The section 360 is marked with a longitudinal line 362 of a solid color distinguishable from the background color of the filament. The longitudinal line 362 serves to identify section 360 but, standing alone, does not indicate the orientation of the retainers 364 within the section 360. The longitudinal orientation of line 362 is readily distinguishable from the bands 302 of section 300. The different orientation of line 362 and bands 302 may be visually observed and thus allows the physician to distinguish section 360 from section 300. Longitudinal lines, such as line 362, but having different thickness, patterns or colors, may also be used to distinguish one section of filament from another. of different thickness

FIG. 3H shows a section 370 of a self-retaining suture filament. The section 370 is marked with a spiral 372 of a solid color distinguishable from the background color of the filament. A spiral may be used to indicate orientation of a filament because a spiral may be left-handed or right-handed. The different handedness of the spiral may be visually observed and thus allow the physician to determine the orientation of the retainers. The handedness of the spiral 372 may however be more difficult to discern and harder to associate with a particular orientation of retainer than for example a simple shape such as shown in FIGS. 3D and 3E

FIG. 3I shows a section 380 of a self-retaining suture filament. Section 380 is marked with typographical characters (such as "R" for right and "L" for left). Characters such as alphanumeric characters 382 and arrows 384 may be used to identify sections of suture and the orientation of the retainers in that section if they are observable by the physician. However, under many circumstances the suture will be too fine for such markers to be discernible by the physician with the level of magnification available utilized during the surgical procedure.

### D. Visible/ Recognizable Needle/Pledget Markers

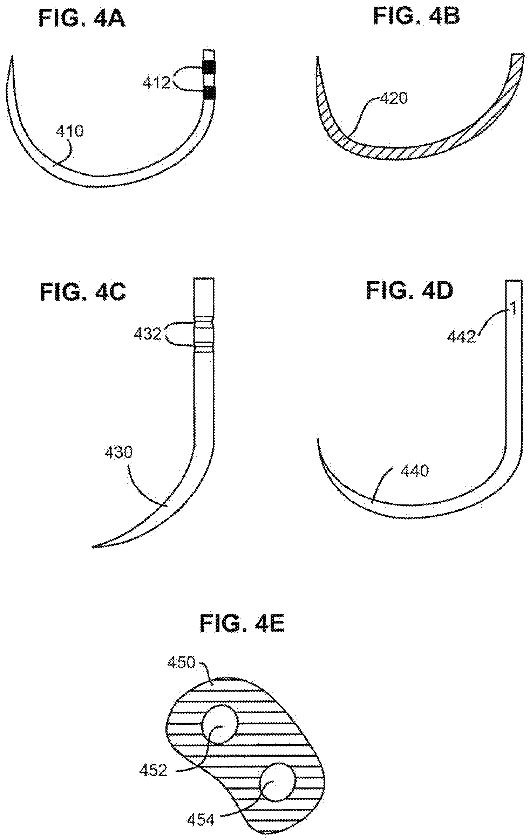

As described above, the needle or another object associated with a section of a heterofunctional suture filament may be marked to enable that section to be identified and distinguished from other sections instead of, or in addition to, marking the suture filament itself. FIGS. 4A-4E illustrate alternative markers that may be placed upon needles and/or pledgets of self-retaining suture systems. It is to be understood that such markers, and, in fact, some other markers described herein, if desired, can be present as an image in the visible light wavelength range or in the non-visible wavelength range. In the non-visible (but otherwise recognizable) wavelength range, a detector would be used to located and image the non-visible marker so that the doctor would have the use and benefit of this marker.

FIG. 4A shows a semicircular needle 410 having two bands 412 with different visual/recognizable characteristics than the remainder of the surface of needle 410. The bands 412 may be colored sections, or sections treated to reduce or change reflection of light. A different number, color or placement of bands may be used to differentiate one needle from another. The bands 412 may, for example be printed sections of the needle surface, or oxidized sections of the needle surface.

FIG 4B shows a compound curve needle 420. The surface of needle 420 exhibits a different visual characteristic that the surface of e.g. needle 410. Needles typically have the reflective silver color characteristic of surgical steel. Different visual characteristics may be achieved by using a different metal, or by coloring, treating or changing the reflectivity of the steel needle in some way such as oxidation, heat treatment and the like. Then one needle can be silver in color and the other needle can be black in color.

FIG. 4C shows a quarter-circle needle 430. Needle 430 has two circumferential grooves 432 which can be observed on the shank of the needle. A different number, size, or placement of grooves may be used to differentiate one needle from another. Other structural features may be cut into or added onto the surface of the needle by processes such as for example engraving or stamping so long as they features can be differentiated and do not interfere with the function of the needle.

FIG. 4D shows a J-shaped needle 440. Needle 440 is marked with an alphanumeric character 442. Needles may be marked with such characters so long as the characters may be discerned and differentiated by the physician. The characters may be marked on the needle using any of the above techniques.

FIG. 4E shows a pledget 450 which may be associated with a section of suture by passing the filament through holes 452, 454. Pledgets may be marked using any of the means previously discussed to identify and differentiate sections of suture and indicate the orientation of retainers in the section. Pledgets and the like may be permanently attached or removably attached to a suture or section of the suture.

### E. Variable Markers Indicative of a Suture Condition

Visual/recognizable markers may be used for other functions instead of, or in addition to, identifying and differentiating sections of a heterofunctional suture and indicating the orientation of the suture. Markers may also be utilized to indicate other features or conditions of the suture. A marker indicative of a fixed feature such as the material from which the suture is made could be in the form of a color code or the like that provides a visual/recognizable clue to the physician regarding the suture he is using without having to check the packaging. Non-sterile portions of the suture packaging may be removed by a physician's assistant, for example, with the physician observing or not observing the labeling. Thus, a fixed marker associated with the suture may be useful for the physician to confirm that they are using the suture they requested. Again, the marker can provide an image in the visible and/or non-visible light range.

A marker that is indicative of a condition of the suture must undergo a discernible change in appearance or other recognizable characteristic in response to a change in the condition. For example, in endoscopic applications where long instruments are used through ports or even operated remotely, it can be difficult or impossible for the physician to feel the tension applied to the suture by the instruments or by the suture to tissue. To replace the reduced or missing haptic feedback, it is advantageous to provide a variable marker indicative of the tension in the suture. The variable marker provides a visual or recognizable cue that can be observed by the physician through the endoscope (or recognized by other means).

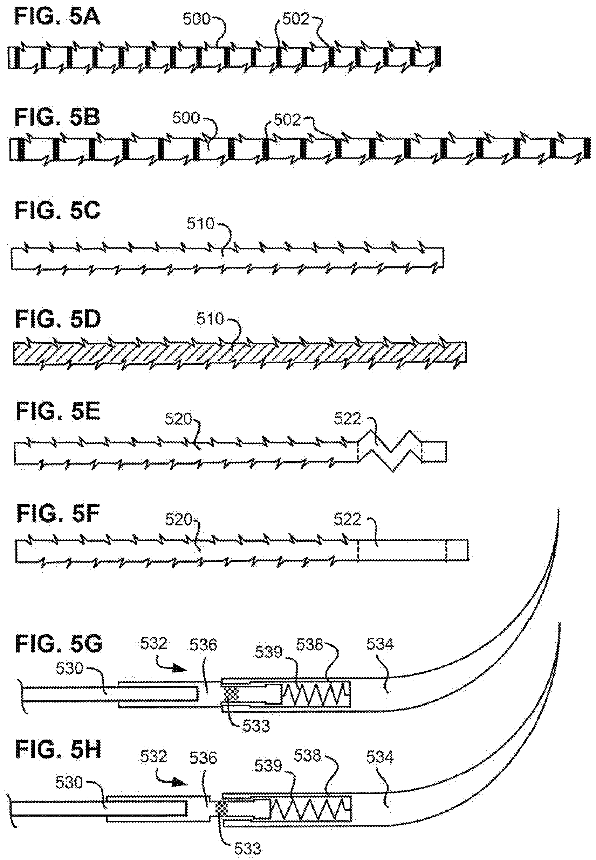

In a simple case, as shown in FIGS. 5A and 5B, a self-retaining suture filament 500 is provided with a plurality of bands 502 spaced at regular intervals along the suture filament. Self-retaining filament 500 is somewhat elastic and therefore stretches in response to tension applied to the suture. Thus, when tension is applied to the suture filament in FIG. 5A, it stretches to the configuration shown in FIG. 5B. However, without bands 502, it may be difficult or impossible for a physician to recognize and/or observe the extension of filament 500. The spacing of bands 502 increases as self-retaining suture filament 500 stretches thereby providing a visual cue to the physician regarding the extension of the filament. Because the stretching of the filament is related to the tension in the filament, the spacing of the bands provides the physician with a visual cue as to the tension in the filament. Note that in this case, the markers do not themselves change, and the change in spacing of the bands is dependent upon the elongation of the suture filament. Additionally, the band could partially cover a retainer and partially cover the suture filament. Upon stretching of the suture, the filament would stretch, and the retainer would not causing misalignment between the band portion on the retainer and the band portion on the filament. This misalignment would indicate that the filament was in tension.

An alternative self-retaining suture filament 510 is shown in FIGS. 5C. and 5D. Filament 510 is provided with a variable marker that changes a visual state in response to tension in suture filament 510. The marker may be a surface marker or may be a feature of the material of which filament 510 is made. Moreover, the marker may cover the entirety of the filament or may be restricted to particular sections of the filament. As shown in FIGS. 5C and 5D, when tension is applied to filament 510 the marker changes from the visual appearance of FIG 5C (illustrated as white) to the visual appearance of FIG. 5D (illustrated as shaded). The change in visual appearance may be a change in color, light transmission, light reflectivity etc. The change in visual appearance may be a change in appearance not observable by the naked eye, but that can be visualized using the endoscope or another visualization system in use. Such a change can include a change in light polarization, or optical properties at wavelengths invisible to the naked eye but observable using a suitably configured video camera.

For example, markers can be made of two colors that under stress blend and present a third color or various intensities of shades of a different color depending upon the degree of stress. The two different colors can be placed at different depths of a suture and when the suture is stressed the colors blend or overlap each other to present different colors. The different depths of the suture can be made of different materials that stretch to different degrees when the suture is stressed and thus the different colors at different depths would blend or overlap each other to present a different color. The different levels can be co-extruded to provide, as desired, each layer with different types of suture material and different colored markers. Further the different colored markers can be placed side by side on the surface of the suture and when the suture is under stress the colors blend or overlap or become differently oriented relative to each other such that they present a different color or shade and the color or shade may be dependent upon the degree of stress that the suture is under. Instead of different colors, different patterns can be used and then the patterns overlap or otherwise combine due to stress being placed on the suture, a different pattern such as an interference or interfering pattern can be presented.

An alternative self-retaining suture filament 520 is shown in FIGS. 5E, 5F. Filament 520 is provided with a variable marker in the form of a mechanical feature 522 which undergoes a visible physical change in response to the application of tension on the suture. As shown in FIGS. 5E and 5F, when tension is applied to filament 520 the mechanical feature 522 changes from the configuration in FIG 5E to the configuration of FIG. 5F. The change in structural appearance is selected such that it is observable by the physician under the conditions in which the suture is used. The change in structural appearance may be a change in appearance not observable by the naked eye but that can be visualized using the endoscope or other visualization system in use. Preferably the change in structural appearance does not interfere with the function of the suture.

As shown in FIGS. 5G and 5H, a variable marker may form part of a needle or other device fixed to the suture filament rather than being part of the filament itself. As shown in FIGS. 5G and 5H, a mechanical sensor 532 is included in a needle 534 to indicate the tension applied to a filament 530. Mechanical sensor 532 includes a cap 536 swaged to filament 530, cap 536 is received in a cavity 538 of needle 534. A device, such as a spring 539, that elongates in response to tension is positioned between the cap 536 and the end of the cavity 538. When tension is applied to the filament 530, spring 539 extends, and cap 536 slides out of cavity 538 revealing a marker 533 that was previously concealed inside cavity 538. The spring constant of spring 539 is selected such that visual indicator becomes visible when a desired tension is reached. A different spring may be used for sutures, and/or applications requiring a different maximum tension. The mechanical sensor 532 is indicative the tension applied to the suture by the needle. Similar mechanical sensors may be attached in line with the suture filament at other locations where an indication of tension is desired. Instead of a spring, the filament 530 could be directly connected to the needle in cavity 538. As the filament stretches, the marker 533 would become extended from the cavity 538 as seen in Fig. 5H. Still further, the above embodiment could be located in the suture body where a marker extends from a cavity or from under a sleeve of a suture body when the suture is stressed.

Other passive mechanical sensors may indicate suture properties through means other than displaying a visible marker or optically detectable marker. For example, a wireless passive strain sensor may be incorporated into a suture or a needle or another device associated with the suture to allow remote sensing of the tension using a non-optical sensor. One such sensor is disclosed in Tan et al., "A wireless, passive strain sensor based on the harmonic response of magnetically soft materials" Smart Materials And Structures 17:1-6 (2008). Tan et al. disclose a sensor made of a ferromagnetic sensing element separated by a deformable elastic material from a permanent magnetic strip. Adjusting the strain applied to the sensor changed the distance between the sensor and the permanent magnet by deforming the elastic material. This change in distance created a detectable change in the harmonic response of the sensor to an alternating magnetic field. Thus a simple passive sensor provides a strain signal that can be detected remotely using an external magnetic field. Similarly passive strain sensors associated with the suture or needle can be read using other remote sensing technologies, such as ultrasound, fluoroscopy, and the like.

### F. "Active" Suture

Fixed or variable markers for suture filaments may also be provided by active systems instead of, or in addition to, other marking methods. Such active visible markers may be utilized to indicate features or conditions of the suture.

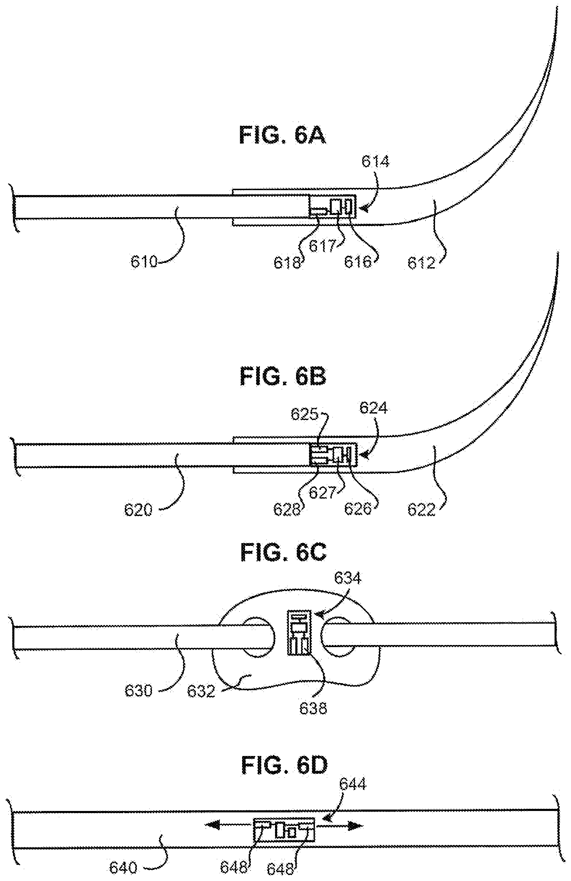

FIG. 6A shows one simple example of an active visible suture marker. As shown in FIG. 6A, a suture filament 610 is swaged to a needle 612. An active marker system 614 is disposed inside the needle 612 adjacent suture filament 610. Active marker system 614 comprises a power source 616, a light controller 617 and a light source 618. Light source 618 is positioned adjacent suture filament 610 such that light from light source 618 may be directed into suture filament 610 and observed by the physician. The light signal will be visible within the suture filament along the length of the suture filament and will attenuate as it passes down the filament away from the light source depending upon the characteristics of the filament.

Light source 618 is controlled by light controller 617 which takes power from power source 616. Light controller 617 determines the characteristics of the light signal provided by light source 618. The light signal may be varied over time such as by turning it on, turning it off and/or flashing at different speeds. The light signal may also be varied in color if light source 618 is capable of producing light of different wavelengths. If power source 616 is a battery, the available power will limit the time that light source 618 may be operated. Thus, it will be desirable that light source 618 be activated by light controller 617 only at the beginning of the procedure utilizing suture filament 610. The activation may be achieved using a magnetic switch, mechanical switch, electromagnetic sensor or the like. In one embodiment, the light source can be an LED and the controller a sensor that measures stress, strain or tension. The LED and sensor can be made in a semiconductor chip. The power supply can be passive, such as in a passive RFID tag. Then a source of radiation may activate the RF power source to power the sensor and the LED light source. In some embodiments the sensor may comprise an accelerometer.

In a dual-armed suture system, another active marker system 614 may be provided in another needle swaged to the opposite end of suture filament 610. Where two active marker systems 614 are used, they may be differentiated based upon the characteristics of the light signals provided by the light source 618. For example, each light source may be controlled to provide light of a different wavelength than the other. Alternatively, one light source may provide a constant light signal whereas the other light source may provide a flashed light signal. If light signals from both light sources overlap in the suture filament, then the light signals may be attenuated by adjusting the power of the light signals, reducing the light transmission of the suture filament or placing a barrier such as an opaque section to block light transmission between one section of suture filament 610 and another section of suture filament 610.

An active marker indicative of a feature, such as the material of which a section of a heterofunctional suture is made, or the orientation of retainers in a particular section of a self-retaining suture, can be in the form of a color code or the like that provides a visual cue to the physician regarding the suture he is using without having to check the packaging. Non sterile portions of the suture packaging may be removed by a physician's assistant for example with the physician observing the labeling. Thus an active visible marker associated with the suture may be useful for the physician to confirm they are using the suture they requested.

Active visual markers may also be utilized to indicate conditions of the suture. An active visual marker that is indicative of a condition of the suture undergoes an observable change in appearance in response to the condition. Thus, in one example a light signal may be modulated in response to tension in a suture providing a visual cue that can be observed by the physician. FIG. 6B shows one simple example of an active visible suture marker indicative of a variable condition. As shown in FIG. 6B, a suture filament 620 is swaged to a needle 622. An active marker system 624 is disposed inside the needle 622 adjacent suture filament 620. Active marker system 624 comprises a power source 626, a light controller 627 and a light source 628. Active marker system 624 also includes a sensor 625 for monitoring a condition of suture filament 620. Light source 628 is controlled by light controller 627 which takes power from power source 626. Light controller 627 controls the characteristics of the light signal provided by light source 628. The light signal may be varied over time such as by turning it on, turning it off and/or flashing at different speeds. The light signal may also be varied in color if light source 628 is capable of producing light of different wavelengths.

The light signal provided by light source 628 is modulated by controller 627 in response to the output of sensor 625. Thus the light signal provided by light source 628 is modulated in response to the condition monitored by sensor 625. For example, sensor 625 may be a force sensor, such as an accelerometer, and controller 627 (also part of the accelerometer, for example, in this embodiment) may control light source 628 so that no light signal is provided to suture filament 620 until the sensor indicates that a threshold tension in filament 620 has been achieved. When the tension passes the threshold then controller 627 turns on light source 628 providing a cue to the physician. Alternatively, the light source may be on initially and then flashed when the tension reaches the threshold.

As shown in FIG. 6C, an active marker system need not be part of a needle. An active marker system 634 may, for example, be provided in a pledget 632 or another device attached to or associated with suture filament 632. The active marker system 634 may provide a light signal utilizing suture filament 630, pledget 632 or directly from light source 638 to the physician. Alternatively, as shown in FIG. 6D, some, or all of an active suture marker system 644 may be miniaturized and embedded within a suture filament 640.

Where an active marker system is provided other than at the end of a suture filament as shown in FIGS. 6C and 6D, the active marker system may advantageously provide two light signals - a first signal indicating the suture filament in a first direction from the active marker system and a second signal indicating the suture filament in a second direction. The two light signals may, for example, be provided by two light sources, one source pointing in each direction along the filament from the active marker system. Thus, as shown in FIG. 6D, an active marker system 644 embedded in a suture filament 640 may include two light sources 648 pointing in opposite directions along suture filament 640 as shown.

### G. Machine-Readable Markers

As described above, a suture filament may be provided with visible markers indicative of features or conditions of the suture filament and/or indicative of particular sections of a suture filament having different features. Such markers are observed by the physician under the operative conditions - which may include e.g. magnification in microsurgical procedures and video display - including wavelength translation/enhancement in endoscopic procedures. However, where machines are available to read, scan and decode suture markers, such markers need not be visible markers or markers that may be visually decoded. The markers may be designed to be machine-readable instead of, or in addition to, being directly visualizable by the physician. While visible markers may be identified and decoded utilizing a video tracking and analyzing system, different visible markers may be more suitable for machine recognition. Moreover, non-visible suture markers or coding may also be used by a computer system to identify sections and conditions of sutures. When the suture sections and/or conditions have been recognized and assessed by the system, information about the sections and conditions of the suture may be provided to the physician by the system. The information may be provided to the physician over any available display system, including a visual, aural or haptic display.

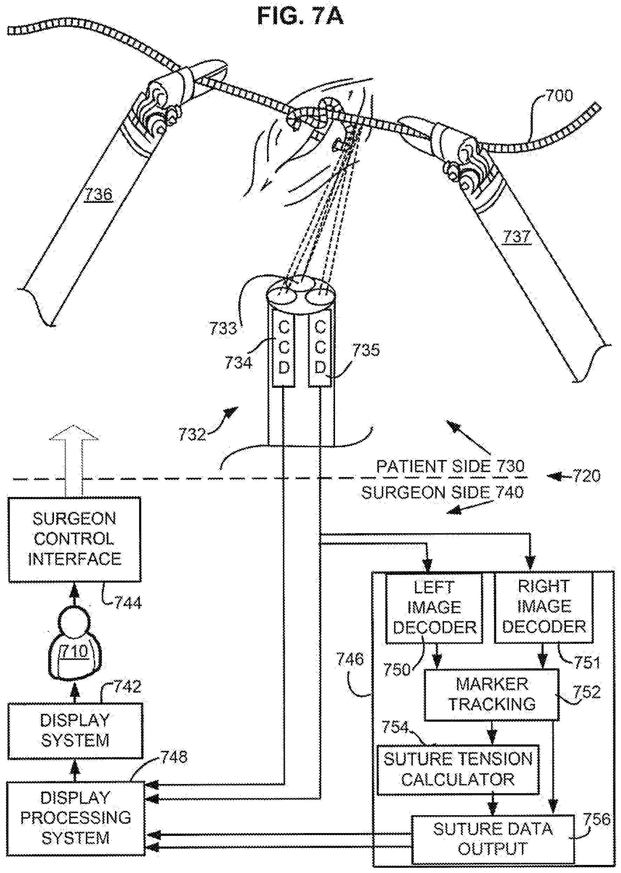

FIG. 7A show features of an endoscopic surgical system (generally) 720 which utilize machine-readable markers to identify sections and conditions of a suture filament 700. Endoscopic surgical system 720 comprises a patient-side system 730 which interfaces with the patient at the operative site and a physician-side system 740 which interfaces with the physician 710. The patient-side system 730 includes an imaging system such as an endoscope, fluoroscope, ultrasound or the like and one or more surgical effectors, such as surgical instruments, catheters, and the like. The physician-side system 740 includes a display system 742 such as video screen for providing information to the physician 710 and a control system 744 such as a joystick or the like for allowing the physician to control the one or more surgical effectors. The control system 744 may be a simple mechanical connection to the surgical instruments.

As shown in FIG. 7A, the patient-side system 730, comprises an endoscope 732 having a light output 733 and two imaging devices in the form of CCDs 734, 735 and associated optics for imaging the operative field. CCDs 734, 735 are separated by the width of endoscope 732 and thus provide a binocular image comprising two separate views of the operative filed. These two views may be utilized to generate a three-dimensional image of the operative field and also to generate three-dimensional tracking data for objects and markers which can be identified in each image. The patient-side system 730 also includes two surgical instruments 736, 737 for manipulating suture filaments, needles and tissue. Surgical instruments 736, 737 are shown as endoscopic instruments and may be manually-operated or servo-operated. Surgical instruments 736, 737 may be forceps, needle drivers, cautery, graspers and/or any of the wide range of surgical instruments available as suited to a particular procedure.

As shown in FIG. 7A, the physician-side system 740 comprises a display system 742 which provides information to physician 710. The display system 742 may include visual, audio and haptic display components. The physician-side system 740 also comprises a physician control interface 744 for allowing physician 710 to control the system including controlling movement of surgical instruments 736, 737. The physician control interface 744 may include a keyboard, mouse, joysticks or the like. The physician control interface 744 may include force feedback controllers which also serve to provide information to the physician as part of the physician display system. The physician-side system also includes a suture information system 746 which receives information from the endoscope and decodes it to generate information about the suture. The physician-side system also includes a display processing system 748 for receiving image information from endoscope and integrating it with other information for display to physician 710 by display system 742.

As shown in FIG. 7A, suture information system 746 receives image data from CCDs 734, 735 of patient side system 730. Suture information system 746 includes a left image decoder 750 which receives and decodes image data from CCD 734 and right image decoder 751 which receives and decodes image data from CCD 735. Image decoders 750, 751 identify suture markers in the video image data received from a CCD of 734, 735 of endoscope 732. Marker tracking system 752 receives marker identity and position data from image decoders 750 and 751. The position of a marker will differ slightly between the left image and the right image. From the difference in positions, marker tracking system 752 can identify the location of the markers in three dimensions. In one embodiment, suture information system 746 includes a suture tension calculator 754 which calculates the tension in the suture from the marker tracking information. The suture tension information and marker tracking information is provided to suture data output system 756. Suture data output system 756 provides the information to display processing system 748 which integrates the suture data with the other information received form the patient-side system.

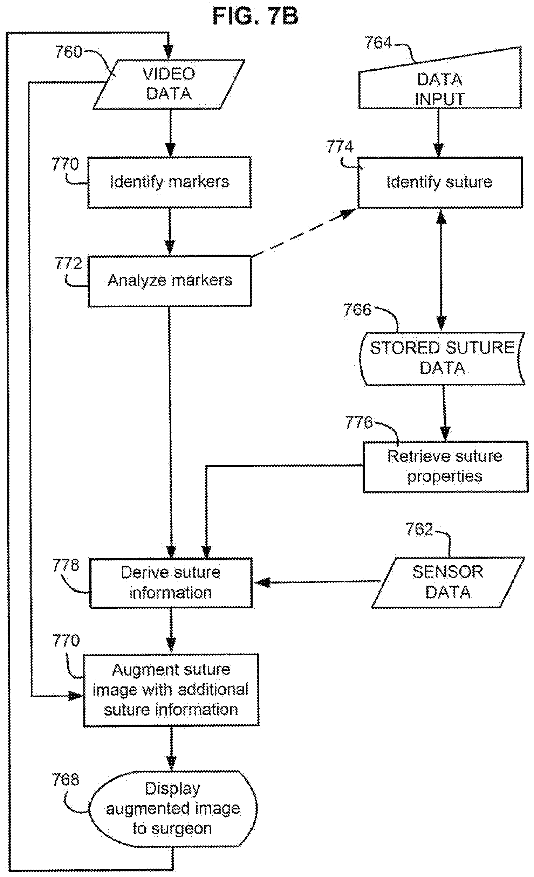

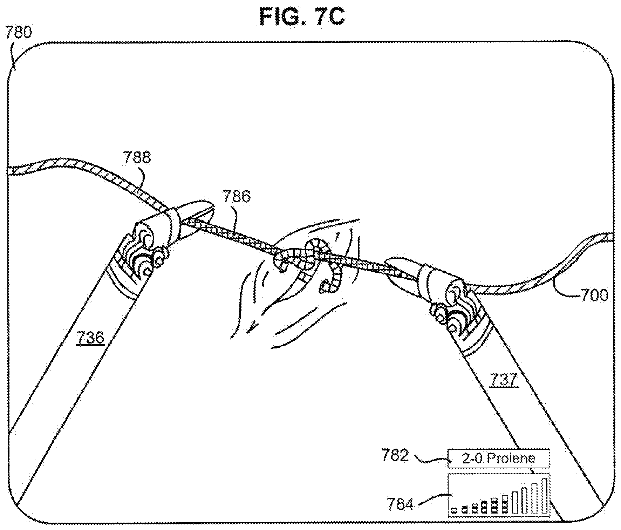

In the embodiments of Fig 7A, 7B, and 7C the system and for example, the suture tension calculator, in addition to causing a visual, audio or haptic or other alarm signal to be generated and sent to the physician can place limits on the maximum amount of stress which can be placed on the suture. Further as each type of suture has different characters, the system can use a database to identify the amount of stress that any type of suture can be placed under and can cause the system to limit the amount of stress that the doctor can place on the suture depending upon the type of suture being deployed in the patient during that procedure. Further the system can cause various levels or intensities of sound, light, or haptic feedback to occur depending on the amount of stress on the suture and the type of suture being deployed at the time in the patient. Again the various characteristic of each suture can be stored in a database in the system and the system can recognize the type of suture being deployed and adjust the feedback and/or the maximum stress the suture can be placed under.

FIG. 7B is a flow chart illustrating general process steps for operating a system utilizing machine-readable suture markers or active suture markers. In the flow chart, various algorithmic steps are summarized in individual "blocks". Such blocks describe specific actions or decisions to be made or carried out as the algorithm proceeds. Where a microcontroller (or equivalent) is employed, the flow charts presented herein provide the basis for a "control program" that may be used by such a microcontroller (or equivalent) to effectuate the desired control of the device. Those skilled in the art may readily write such a control program based on the flow charts and other descriptions presented herein.

As shown in FIG. 7B a system receives data input in the form of video data 760. The system may also receive additional sensor data 762 indicative of suture conditions. The sensor data 762 may be data transmitted to the system by sensor devices such as shown in FIGS. 6A-6C. Sensor data 762 includes all data received by the system from the suture or sensors associated with the suture. The system may also receive data input 764 such as data from a keyboard, mouse, barcode or the like. Data input 764 may include, for example, an identification of the suture filament by entering a product identifier, reading a barcode or the like. As different sutures will have different features, the system will also have access to stored suture data 766 which may be a database of suture information identifying different sutures, the features of the sutures, and the markers of the sutures. The stored suture data 766 may be stored within the system or accessed over a network.

Referring again to FIG. 7B, at step 770, the system receives video data 760 and identifies markers present in the video data. At step 772, the system analyzes the markers identified in step 770 to generate information about the suture such as identifying sections of the suture, orientation of sections of suture, location of the suture and condition of the suture, such as tension. At step 774 the system identifies the suture based upon the data input 764. Alternatively, the suture may be identified based upon analysis of the markers at step 772 if the markers are characteristic of the suture (such as e.g. a barcode). The identity of the suture is utilized to identify the stored suture data about the features of the suture. This may include the manufacturer, size and material of the suture, the strength of the suture, and also information regarding correlations between markers of the suture and particular features of the suture and/or sections of the suture. For example, the stored data may indicate that a particular suture in use has a maximum strength of 7 pounds and an elongation of 1% per pound. These features are retrieved at step 776 and then used at step 778 to derive further information about the suture. For example, the system may analyze the marker positions to determine the elongation of the suture from the video data 760. From the elongation of the suture or a section of suture, the system can determine the tension in the suture using the stored suture data. The system may also compare the calculated suture tension with the maximum suture tension using the stored suture data. In some case the system may then issue a warning if the calculated suture tension over a threshold (which may be a predetermined percentage of the maximum tension). At step 770 the system augments the suture image data with additional suture information. The augmented image including the suture information is displayed to the physician at step 768. The system then loops back to receive more video data 760 and update the augmented display at step 768.

Display processing system 748 of FIG. 7A may integrate the suture data output into other display data in many different ways. See step 770 of FIG. 7B. Suture tension data may be displayed to the physician as haptic, visual or audio feedback. For haptic feedback, the calculated tension may be displayed as force-feedback to a force-feedback controller. For audio feedback, a sound may be played to the physician indicative of the tension in the suture. The fixed sound may be played when a particular desirable suture tension is reached or exceeded. Alternatively, a variable sound may be used which, for example, increases in frequency of occurrence, pitch or volume as tension in the suture increases. For visual feedback, a value, symbol or color indicative of suture tension may be displayed to the physician as part of or adjacent to the video display of the operative field. In some cases the visual data may be added to the image of the operative field by, e.g., superimposing a value, symbol or color on the image of suture 700 or surgical instruments 736, 737. Thus, display system 742 provides physician 710 with an augmented display that includes a representation of the operative filed augmented with information derived from the suture markers using suture information 746.

FIG. 7C illustrates an augmented video display 780 of the patient-side system 730 of FIG. 7A. FIG. 7C illustrates a number of ways in which a visual display may be augmented to indicate features and/or conditions of the suture. The type and size of suture 782 may be overlayed on the display. A tension meter 784 may be used to show the maximum tension and the actual tension in relative terms. Alternatively, actual tension and maximum tension information may be provided in absolute terms. Tension or other conditions of the suture may also be registered with and overlayed on the image of the suture and or tools. For example, the system can overlay the suture with a different color based upon a feature of the suture (such as e.g. retainer orientation or tension) derived from marker data or other data. Thus, as shown in FIG. 7C, the section of suture 700 between instruments 736, 737 is highlighted with color 786 representative of tension in suture 700. Whereas sections of suture 700 not tensioned by tools 736, 737 are highlighted with a different color 788 (or not highlighted).

### H. Tactile Suture Markers

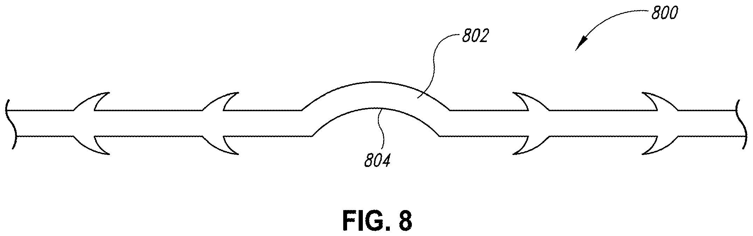

Where a surgical procedure is being performed manually or by sufficiently sensitive robotically-assisted means, tactile markings may be provided; these may be particularly useful in sutures that are not self-retaining and in the retainer-free sections of self-retaining sutures. For example, in the case of self-retaining sutures, the doctor may wish to identify the transition segment; accordingly, the such as the transition segment may be provided with a configuration that is more easily detectible by touch than the simple absence of retainers, thereby obviating the surgeon's need to repeatedly and/or vigorously feel along the suture body to locate the transition segment. Such tactile markings may be provided in any section of interest of a suture, and may include a deformation in the section of interest, such as deformation 804 of transition segment 802 in suture 800 shown in FIG. 8. This may be effected by introducing a fold in the suture transition segment to impair a deformation which can be easily felt (and indeed may also be visible). Other types of tactilely-detectible suture deformations may similarly be provided.

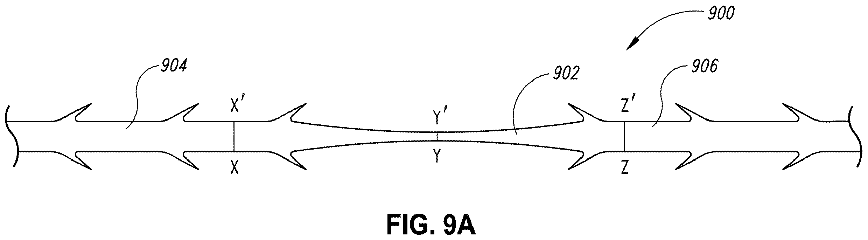

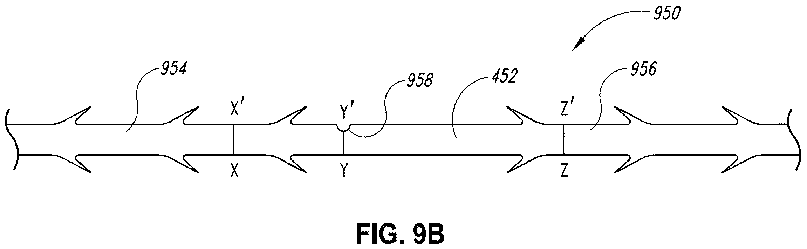

Easily detectible indentations in the section of interest may also be provided, such as by compression (e.g., stamping) of all or part of the section to deform the suture material therein, and by removal of suture material (e.g., cutting, etching, abrading, laser removal) from all or part of the suture section of interest to create an indentation. Indentations may take any form (such as, without limitation, grooves, wells, dents), as long as the diameter of the suture body at the indentation is less than the diameter of the suture body elsewhere, and as long as the diameter of suture body at the indentation remains sufficient to withstand breakage from forces normally exerted during suture deployment and engagement. For example, an indentation or narrowing of the suture diameter at the transition segment may assist a doctor in locating the transition segment. Referring to FIG. 9a, bidirectional self-retaining suture 900 includes first plurality of retainers 904, second plurality of retainers 906, and transition segment 902 (which may or may not correspond to the actual mid-point of the suture, depending on the arrangement of retainers). The diameter of suture 900 narrows gradually in transition segment 902 so that at position y and y', the diameter is less than the diameter at either of positions x and x' or and z and z'. In FIG. 9B, a more abrupt indention 958 is provided in transition segment 952 between retainer pluralities 954 and 956 of suture 950. Again, the diameter of the suture at position y to y', where the depth of the indentation 958 is at its greatest, is less than the diameter at either of positions x and x' or and z and z'.

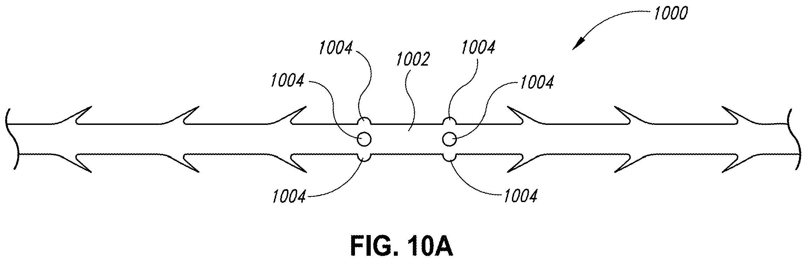

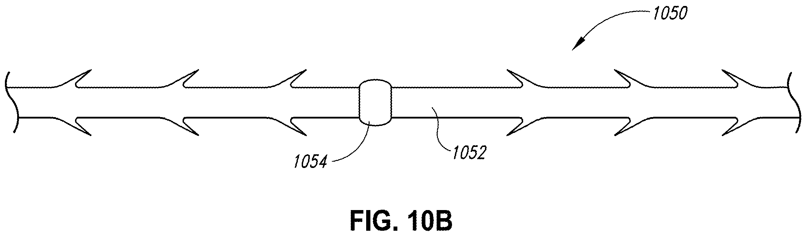

Other examples of tactile suture markings include a tactilely detectible increase in the diameter of the suture body at all or part of the section of interest. For example, protrusions on the surface of the section of interest that are easily detectable and distinguishable from tissue retainers may assist a surgeon in locating the section of interest, such as those protrusions 1004 in transition segment 1002 on suture 1000 in FIG. 10a. Alternatively, a ridge may be provided which runs continuously or discontinuously around the circumference of the suture along all or part of the section of interest , or such a ridge may run along only a portion of the circumference of the section of interest; see, for example, ridge 1054 in transition segment 552 of suture 550 in FIG. 10b. Similarly, other types of relief configurations or forms (such as filamentous projections, squares or other shapes) may be provided on the circumference of the section of interest. It should be noted that while such relief forms should be tactilely easily differentiable from tissue retainers, it is not necessary that they be configured to avoid inducing a fibrotic response in the tissue. Such increases in suture diameter at the section of interest can be created during manufacture of the suture thread (by, for example, extruding a larger amount of suture material for a particular length of a suture thread during an extrusion manufacturing process, cutting or stamping a suture thread with an expanded diameter portion, and so forth), or after the manufacture of the suture thread (by, for example, cutting material away from the ends of a suture thread, adding material to the desired portion of the thread by, for example, polymerisation, and so forth).

Other forms of tactile demarcations include texture differences. Providing texture differences to all or part of the transition segment includes providing a plurality of areas of increased and/or decreased suture body diameter in the section of interest. For example, a plurality of indentations, a plurality of relief configurations, and any combinations thereof may be provided in the section of interest, by methods including, without limitation, compression, cutting, coating, application of agents such as abrasives, polymerisers, acid etchants, base etchants, and so forth.

### I. Materials

Suture threads described herein may be produced by any suitable method, including without limitation, injection molding, stamping, cutting, laser, extrusion, and so forth. With respect to cutting, polymeric thread or filaments may be manufactured or purchased for the suture body, and the retainers can be subsequently cut onto the suture body; the retainers may be hand-cut, laser-cut, or mechanically machine-cut using blades, cutting wheels, grinding wheels, and so forth. During cutting either the cutting device or the suture thread may be moved relative to the other, or both may be moved, to control the size, shape and depth of cut 210. Particular methods for cutting barbs on a filament are described in U.S. Patent Application Publication N° US2003/041426 A1 titled "Method Of Forming Barbs On A Suture And Apparatus For Performing Same" to Genova et al., and U.S. Patent Application Publication N° US2004/060410 A1 titled "Barbed Sutures" to Leung et al. The sutures may be made of any suitable biocompatible material, and may be further treated with any suitable biocompatible material, whether to enhance the sutures' strength, resilience, longevity, or other qualities, or to equip the sutures to fulfill additional functions besides joining tissues together, repositioning tissues, or attaching foreign elements to tissues.

Additionally, self-retaining sutures described herein may be provided with compositions to promote healing and prevent undesirable effects such as scar formation, infection, pain, and so forth. This can be accomplished in a variety of manners, including for example: (a) by directly affixing to the suture a formulation (e.g., by either spraying the suture with a polymer/drug film, or by dipping the suture into a polymer/drug solution), (b) by coating the suture with a substance such as a hydrogel which will in turn absorb the composition, (c) by interweaving formulation-coated thread (or the polymer itself formed into a thread) into the suture structure in the case of multi-filamentary sutures, (d) by inserting the suture into a sleeve or mesh which is comprised of, or coated with, a formulation, or (e) constructing the suture itself with a composition. Such compositions may include without limitation anti-proliferative agents, anti-angiogenic agents, anti-infective agents, fibrosis-inducing agents, anti-scarring agents, lubricious agents, echogenic agents, anti-inflammatory agents, cell cycle inhibitors, analgesics, and anti-microtubule agents. For example, a composition can be applied to the suture before the retainers are formed, so that when the retainers engage, the engaging surface is substantially free of the coating. In this way, tissue being sutured contacts a coated surface of the suture as the suture is introduced, but when the retainer engages, a non-coated surface of the retainer contacts the tissue. Alternatively, the suture may be coated after or during formation of retainers on the suture if, for example, a fully-coated rather than selectively-coated suture is desired. In yet another alternative, a suture may be selectively coated either during or after formation of retainers by exposing only selected portions of the suture to the coating. The particular purpose to which the suture is to be put or the composition may determine whether a fully-coated or selectively-coated suture is appropriate; for example, with lubricious coatings, it may be desirable to selectively coat the suture, leaving, for instance, the tissue-engaging surfaces of the sutures uncoated in order to prevent the tissue engagement function of those surfaces from being impaired. On the other hand, coatings such as those comprising such compounds as anti-infective agents may suitably be applied to the entire suture, while coatings such as those comprising fibrosing agents may suitably be applied to all or part of the suture (such as the tissue-engaging surfaces). The purpose of the suture may also determine the sort of coating that is applied to the suture; for example, self-retaining sutures having anti-proliferative coatings may be used in closing tumour excision sites, while self-retaining sutures with fibrosing coatings may be used in tissue repositioning procedures and those having anti-scarring coatings may be used for wound closure on the skin. As well, the structure of the suture may influence the choice and extent of coating; for example, sutures having an expanded segment may include a fibrosis-inducing composition on the expanded segment to further secure the segment in position in the tissue. Coatings may also include a plurality of compositions either together or on different portions of the suture, where the multiple compositions can be selected either for different purposes (such as combinations of analgesics, anti-infective and anti-scarring agents) or for their synergistic effects.

### J. Clinical Uses

In addition to the general wound closure and soft tissue repair applications, self-retaining sutures can be used in a variety of other indications.

Self-retaining sutures described herein may be used in various dental procedures, i.e., oral and maxillofacial surgical procedures and thus may be referred to as "self-retaining dental sutures." The above-mentioned procedures include, but are not limited to, oral surgery (e.g., removal of impacted or broken teeth), surgery to provide bone augmentation, surgery to repair dentofacial deformities, repair following trauma (e.g., facial bone fractures and injuries), surgical treatment of odontogenic and non-odontogenic tumors, reconstructive surgeries, repair of cleft lip or cleft palate, congenital craniofacial deformities, and esthetic facial surgery. Self-retaining dental sutures may be degradable or non-degradable, and may typically range in size from USP 2-0 to USP 6-0.

Self-retaining sutures described herein may also be used in tissue repositioning surgical procedures and thus may be referred to as "self-retaining tissue repositioning sutures". Such surgical procedures include, without limitation, face lifts, neck lifts, brow lifts, thigh lifts, and breast lifts. Self-retaining sutures used in tissue repositioning procedures may vary depending on the tissue being repositioned; for example, sutures with larger and further spaced-apart retainers may be suitably employed with relatively soft tissues such as fatty tissues.

Self-retaining sutures described herein may also be used in microsurgical procedures that are performed under a surgical microscope (and thus may be referred to as "self-retaining microsutures"). Such surgical procedures include, but are not limited to, reattachment and repair of peripheral nerves, spinal microsurgery, microsurgery of the hand, various plastic microsurgical procedures (e.g., facial reconstruction), microsurgery of the male or female reproductive systems, and various types of reconstructive microsurgery. Microsurgical reconstruction is used for complex reconstructive surgery problems when other options such as primary closure, healing by secondary intention, skin grafting, local flap transfer, and distant flap transfer are not adequate. Self-retaining microsutures have a very small caliber, often as small as USP 9-0 or USP 10-0, and may have an attached needle of corresponding size. The microsutures may be degradable or non-degradable.

Self-retaining sutures as described herein may be used in similarly small caliber ranges for ophthalmic surgical procedures and thus may be referred to as "ophthalmic self-retaining sutures". Such procedures include but are not limited to keratoplasty, cataract, and vitreous retinal microsurgical procedures. Ophthalmic self-retaining sutures may be degradable or non-degradable, and have an attached needle of correspondingly-small caliber.

Self-retaining sutures can be used in a variety of veterinary applications for a wide number of surgical and traumatic purposes in animal health.

Although the present invention has been shown and described in detail with regard to only a few exemplary embodiments of the invention, it should be understood by those skilled in the art that it is not intended to limit the invention to the specific embodiments disclosed. Various modifications, omissions, and additions may be made to the disclosed embodiments without materially departing from the teachings and advantages of the invention, particularly in light of the foregoing teachings. Accordingly, the scope of the invention is defined by the following claims.

## Claims

1. A self-retaining suture (102) comprising a plurality of sections (140; 142; 144; 146; 148) and a plurality of retainers (130) wherein:
at least one section has features different from the features of at least one other section, wherein the features are fixed properties of the suture, such as material, retainer configuration, nominal diameter, or needle configuration, and
said at least one section has a plurality of markers (152; 154; 156) which allows it to be distinguished from said at least one other section,
wherein the markers are non-visible and are configured to be read, scanned and decoded by a machine.

2. The self-retaining suture of claim 1, wherein the suture comprises a biodegradable material, and/or a coating, particularly wherein the coating comprises at least one composition selected from the class consisting of anti-proliferative agents, anti-angiogenic agents, anti-infective agents, fibrosis-inducing agents, anti-scarring agents, lubricious agents, echogenic agents, anti-inflammatory agents, cell cycle inhibitors, analgesics, and anti-microtubule agents.

3. The self-retaining suture claim 1, wherein one section comprises a pledget or further comprising a needle (110, 112), particularly wherein the needle comprises an active marker system, more particularly wherein the active marker system comprises a power source, a light controller, and a light source.

4. The self-retaining suture of claim 1, wherein the marker indicates:
an identifying feature of the suture; or
an identifying feature of the section; or
force acting on the suture, particularly wherein the force is a mechanical force; or
a condition of the suture, particularly mechanical strain, or chemical degradation.

5. The self-retaining suture of claim 1, comprising a passive variable marker system, particularly wherein:
the passive variable marker system indicates a change in suture condition, particularly wherein the change comprises mechanical strain, or chemical degradation; or
the passive variable marker system comprises a sensor.

6. The self-retaining suture of claim 1, wherein the suture comprises a dental suture, or a microsuture, or an ophthalmic suture, or a tissue repositioning suture.

7. A method of forming a self-retaining suture according to claim 1, said method comprising
providing biocompatible surgical filament material, adding a non-visible marking agent to a portion of the surgical filament material, forming a suture having a plurality of sections wherein a first section has features different from the features of at least one other section and wherein the at least the first section comprises the marking agent; and,
forming a plurality of retainers in the suture.

8. The method of claim 7, wherein the marking agent is selected from the class consisting of ultraviolet spectrum colourants, and fluorescent colourants, and/or further comprising coating the suture at least in part in at least one composition selected from the class consisting of anti-proliferative agents, anti-angiogenic agents, anti-infective agents, fibrosis-inducing agents, anti-scarring agents, lubricious agents, echogenic agents, anti-inflammatory agents, cell cycle inhibitors, analgesics, and anti-microtubule agents.

## Patentansprüche

1. Selbstsicherndes Nahtmaterial (102), umfassend eine Vielzahl von Segmenten (140; 142; 144; 146; 148) und eine Vielzahl von Haltern (130), wobei:
mindestens ein Segment Merkmale aufweist, die sich von den Merkmalen von mindestens einem anderen Segment unterscheiden, wobei die Merkmale fixierte Eigenschaften des Nahtmaterials sind, wie Material, Halterkonfiguration, Nenndurchmesser oder Nadelkonfiguration, und
wobei das mindestens eine Segment eine Vielzahl von Markern (152; 154; 156) aufweist, wodurch es von mindestens einem anderen Segment unterschieden werden kann,
wobei die Merker nicht-sichtbar sind und ausgestaltet sind, um von einer Maschine gelesen, gescannt und decodiert zu werden.

2. Selbstsicherndes Nahtmaterial nach Anspruch 1, wobei das Nahtmaterial ein biologisch abbaubares Material und/oder eine biologisch abbaubare Beschichtung umfasst, wobei die Beschichtung insbesondere mindestens eine Zusammensetzung ausgewählt aus der Klasse bestehend aus antiproliferativen Mitteln, antiangiogenen Mitteln, antinfektiven Mitteln, fibroseinduzierenden Mitteln, gegen Narbenbildung wirkenden Mitteln, Schmiermitteln, echogenen Mitteln, antiinflammatorischen Mitteln, Zellzyklusinhibitoren, Analgetika und Antimikrotubulimitteln umfasst.

3. Selbstsicherndes Nahtmaterial nach Anspruch 1, wobei ein Segment einen Tupfer oder des Weiteren eine Nadel (110, 112) umfasst, wobei die Nadel insbesondere ein aktives Markersystem umfasst, wobei das aktive Markersystem insbesondere eine Leistungsquelle, eine Lichtsteuerung und eine Lichtquelle umfasst.

4. Selbstsicherndes Nahtmaterial nach Anspruch 1, wobei der Marker angibt:
ein Identifizierungsmerkmal des Nahtmaterials; oder
ein Identifizierungsmerkmal des Segments; oder
Kraft, die auf das Nahtmaterial einwirkt, wobei insbesondere die Kraft eine mechanische Kraft ist; oder
einen Zustand des Nahtmaterials, insbesondere mechanische Belastung oder chemischen Abbau.

5. Selbstsicherndes Nahtmaterial nach Anspruch 1, umfassend ein passives variables Markersystem, wobei insbesondere:
das passive variable Markersystem eine Änderung des Nahtmaterialzustands angibt, wobei insbesondere die Veränderung mechanische Belastung oder chemischen Abbau umfasst; oder
das passive variable Markersystem einen Sensor umfasst.

6. Selbstsicherndes Nahtmaterial nach Anspruch 1, wobei das Nahtmaterial ein Dentalnahtmaterial oder ein Mikronahtmaterial oder ein ophthalmisches Nahtmaterial oder ein Nahtmaterial zur Gewebeneupositionierung umfasst.

7. Verfahren zur Bildung eines selbstsichernden Nahtmaterials nach Anspruch 1, wobei das Verfahren umfasst:
Bereitstellen von biokompatiblem chirurgischem Filamentmaterial, Zufügen eines nicht-sichtbaren Markierungsmittels zu einem Anteil des chirurgischen Filamentmaterials, Bilden eines Nahtmaterials mit einer Vielzahl von Segmenten, wobei ein erstes Segment Merkmale aufweist, die sich von den Merkmalen von mindestens einem anderen Segment unterscheiden, und wobei mindestens das erste Segment das Markierungsmittel umfasst; und
Bilden einer Vielzahl von Haltern in dem Nahtmaterial.

8. Verfahren nach Anspruch 7, wobei das Markierungsmittel ausgewählt ist aus der Klasse bestehend aus Färbungsmitteln des Ultraviolettspektrums und Fluoreszenzfärbungsmitteln, und/oder des Weiteren umfassend mindestens teilweises Beschichten des Nahtmaterials mit mindestens einer Zusammensetzung ausgewählt aus der Klasse bestehend aus antiproliferativen Mitteln, antiangiogenen Mitteln, antiinfektiven Mitteln, fibroseinduzierenden Mitteln, gegen Narbenbildung wirkenden Mitteln, Schmiermitteln, echogenen Mitteln, antiinflammatorischen Mitteln, Zellzyklusinhibitoren, Analgetika und Antimikrotubulimitteln.

## Revendications

1. Suture autorétentive (102) comprenant une pluralité de sections (140 ; 142 ; 144 ; 146 ; 148) et une pluralité de dispositifs de retenue (130), dans laquelle :
au moins une section a des caractéristiques différentes des caractéristiques d'au moins une autre section, les caractéristiques étant des propriétés fixées de la suture, telles que le matériau, la configuration des dispositifs de retenue, le diamètre nominal ou la configuration d'aiguille, et
ladite au moins une section comprend une pluralité de marqueurs (152 ; 154 ; 156) qui lui permettent d'être distinguée de ladite au moins une autre section,
les marqueurs étant non visibles et étant configurés pour être lus, scannés et décodés par une machine.

2. Suture autorétentive de la revendication 1, dans laquelle la suture comprend un matériau biodégradable et/ou un revêtement, en particulier dans laquelle le revêtement comprend au moins une composition choisie dans la classe constituée par les agents antiprolifératifs, les agents antiangiogéniques, les agents antiinfectieux, les agents induisant une fibrose, les agents anticicatriciels, les agents lubrifiants, les agents échogéniques, les agents antiinflammatoires, les inhibiteurs de cycle cellulaire, les analgésiques et les agents anti-microtubules.

3. Suture autorétentive de la revendication 1, dans laquelle une section comprend une compresse ou comprenant en outre une aiguille (110, 112), en particulier dans laquelle l'aiguille comprend un système de marqueur actif, plus particulièrement dans lequel le système de marqueur actif comprend une source d'énergie, un contrôleur de lumière et une source de lumière.

4. Suture autorétentive de la revendication 1, dans laquelle le marqueur indique :
une caractéristique identifiante de la suture ; ou
une caractéristique identifiante de la section ; ou
une force agissant sur la suture, en particulier dans laquelle la force est une force mécanique ; ou
un état de la suture, en particulier une contrainte mécanique ou une dégradation chimique.

5. Suture autorétentive de la revendication 1, comprenant un système de marqueur variable passif, en particulier dans laquelle :
le système de marqueur variable passif indique un changement dans l'état de la suture, en particulier dans laquelle le changement comprend une contrainte mécanique ou une dégradation chimique ; ou
le système de marqueur variable passif comprend un capteur.

6. Suture autorétentive de la revendication 1, dans laquelle la suture comprend une suture dentaire, ou une microsuture, ou une suture ophtalmique, ou un suture de repositionnement de tissu.

7. Procédé de formation d'une suture autorétentive selon la revendication 1, ledit procédé comprenant :
la fourniture d'un matériau de filament chirurgical biocompatible, l'ajout d'un agent de marquage non visible à une portion du matériau de filament chirurgical, la formation d'une suture ayant une pluralité de section, une première section ayant des caractéristiques différentes des caractéristiques d'au moins une autre section, et ladite au moins une première section comprenant l'agent de marquage ; et
la formation d'une pluralité de dispositifs de retenue dans la suture.

8. Procédé de la revendication 7, dans lequel l'agent de marquage est choisi dans la classe constituée par les colorants du spectre ultraviolet, et les colorants fluorescents, et/ou comprenant en outre le revêtement de la suture au moins en partie en au moins une composition choisie dans la classe constituée par les agents antiprolifératifs, les agents antiangiogéniques, les agents antiinfectieux, les agents induisant une fibrose, les agents anticicatriciels, les agents lubrifiants, les agents échogéniques, les agents antiinflammatoires, les inhibiteurs de cycle cellulaire, les analgésiques et les agents anti-microtubules.
